(19) **Europäisches Patentamt / European Patent Office / Office européen des brevets**

(11) **EP 4 144 358 A1**

(12) **EUROPEAN PATENT APPLICATION**
published in accordance with Art. 153(4) EPC

(43) Date of publication:
**08.03.2023  Bulletin 2023/10**

(21) Application number: **21796550.8**

(22) Date of filing: **27.04.2021**

(51) International Patent Classification (IPC):
*A61K 36/61* (2006.01)        *A61K 31/045* (2006.01)
*A61P 31/12* (2006.01)        *A61P 31/14* (2006.01)
*A61P 31/04* (2006.01)        *A61P 11/00* (2006.01)
*A61P 11/02* (2006.01)        *A61P 29/00* (2006.01)
*A61P 37/08* (2006.01)        *A61K 9/12* (2006.01)
*A61K 9/72* (2006.01)

(52) Cooperative Patent Classification (CPC):
Y02A 50/30

(86) International application number:
**PCT/CN2021/090137**

(87) International publication number:
**WO 2021/218945 (04.11.2021 Gazette 2021/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.04.2020  CN 202010367580**

(71) Applicant: **Yunnan Baiyao Group Company Limited**
**Kunming, Yunnan 650504 (CN)**

(72) Inventors:
- LIN, Ruichao
  **Kunming, Yunnan 650504 (CN)**
- DONG, Xun
  **Kunming, Yunnan 650504 (CN)**
- CUI, Tao
  **Kunming, Yunnan 650504 (CN)**
- WANG, Jingkun
  **Kunming, Yunnan 650504 (CN)**
- SUN, Min
  **Kunming, Yunnan 650504 (CN)**

(74) Representative: **v. Bezold & Partner Patentanwälte - PartG mbB**
**Ridlerstraße 57**
**80339 München (DE)**

(54) **ANTIBACTERIAL ANTIVIRAL PHARMACEUTICAL COMPOSITION AND APPLICATION THEREOF**

(57)     An antibacterial antiviral pharmaceutical composition, comprising the following raw materials in parts by weight: 4-14 parts of *Herba Pogostemonis,* 9-28 parts of *Rhizoma Atractylodis,* 5-18 parts of *Herba Elsholtzlae*, 11-33 parts of *Folium Artemisiae Argyi,* 0.1-3 parts of *Flos Caryophylli,* and 2-10 parts of *Herba Menthae*, and further comprising natural borneol and pharmaceutical adjuvants. The pharmaceutical composition can be prepared into medicinal volatile oil. The pharmaceutical composition and the volatile oil thereof can be used for resisting bacteria and viruses, clearing heat, reducing fever and/or treating respiratory diseases, and can be applied to the preparation of medicines for resisting SARS-CoV-2.

Figure 7

## Description

## Technical Field

The present application belongs to the technical field of medicine, and specifically relates to an antibacterial and antiviral pharmaceutical composition and application thereof.

## Background Art

Respiratory system diseases are common clinical diseases and frequently-occurring diseases, common in microbial infection, including bacterial, viral, fungal infections, etc., have the characteristics of high incidence, great harm, easy recurrence and increasingly serious drug resistance.

Among them, bacterial infection is a common cause for respiratory diseases, and there are various pathogenic bacteria which cause respiratory bacterial infection, wherein *Pseudomonas aeruginosa, Staphylococcus aureus, Klebsiella pneumoniae* and the like are common. Clinical chronic pulmonary disease includes chronic obstructive pulmonary diseases, bronchial asthma, pulmonary tuberculosis, etc., and rapid deterioration of these disease or even death will be caused once combined with these bacterial infections. Although the chemical drugs represented by antibiotic have obvious curative effect currently, the side effects thereof are severe, it is easy to cause superinfection and drug resistance is becoming more and more serious.

Respiratory tract viruses are a large class of viruses which can invade the respiratory tract and cause local lesions in the respiratory tract, or only use the respiratory tract as an entry portal and mainly cause lesions of respiratory tissues and organs, which are the major pathogens causing acute respiratory tract infection. According to statistics, more than 90% of the acute respiratory tract infection is caused by viruses, and in particular, upper respiratory tract infection is the clinical common diseases and frequently-occurring diseases. Currently, there are more than 10 viruses known to cause acute respiratory tract infection, and these viruses are generally spread through airborne droplets that is an easy route to infection, and they often cause high morbidity and excess mortality, due to lack of effective and persistent immunity. Especially in the high-incidence season and epidemic season of influenza, it often causes complications and severe cases in children and the elderly. Although vaccines can be used for prevention, they are only effective against influenza viruses, and are not effective against other respiratory tract viruses, and duration of immunity is relatively limited. In terms of treatment, so far, no western medicine has been found to have low toxicity and side effects while ensuring good curative effect.

Therefore, there is an urgent need to provide a drug that can effectively resist bacterium and virus, and has good therapeutic effects on respiratory system diseases.

## Summary of Invention

Therefore, the present application provides an antibacterial and antiviral pharmaceutical composition, and use thereof in preparing a medicament for anti-bacteria, anti-virus, clearing heat, reducing fever and/or treating a respiratory disease.

In order to achieve the above objective, the present application uses the following technical solutions:
an antibacterial and antiviral pharmaceutical composition, comprising the following raw materials in parts by weight:
4-14 parts of *Herba Pogostemonis,* 9-28 parts of *Rhizoma Atractylodis,* 5-18 parts of *Herba Moslae,* 11-33 parts of *Folium Artemisiae Argyi,* 0.1-3 parts of *Flos Caryophylli,* and 2-10 parts of *Herba Menthae Haplocalycis.*

*Herba Pogostemonis* resolves dampness with aroma, harmonizes the stomach to check vomiting, dispels summerheat and releases the exterior; *Rhizoma Atractylodis* dries dampness to fortify the spleen, dispels wind and dissipates cold; *Herba Moslae* clears heat and drains dampness, resists bacteria and reduces inflammation; and *Folium Artemisiae Argyi* warms the meridian and dispels dampness, suppresses cough, dispels phlegm and calms panting. *Herba Pogostemonis, Rhizoma Atractylodis, Herba Moslae,* and *Folium Artemisiae Argyi* are combined in the present application, which is good for reducing inflammation, suppressing cough, calming panting, resolving phlegm, clearing heat, enhancing antibacterial and antiviral effects.

*Flos Caryophylli* warms stomach, downbears counterflow, and wets the kidney, and thus is used to moderate the properties of the above medicinal materials and strengthen antibacterial and antiviral effects.

*Herba Menthae Haplocalycis* dispels filth with aroma, and has antiviral, analgesic, antipruritic, and bactericidal effects, which can reduce the foam phlegm in the respiratory tract, enlarge the effective lumen airway, so as to enhance expectorant effect.

Preferably, the antibacterial and antiviral pharmaceutical composition further comprises, in parts by weight, 0.01-0.2 parts of natural borneol.

The natural borneol can connect all orifices, relieve pathogenic fire, and relieve swelling and pain, and the addition thereof can further improve the antibacterial and antiviral effects of the composition.

Further preferably, the antibacterial and antiviral pharmaceutical composition comprises the following raw materials in parts by weight:

8-10 parts of *Herba Pogostemonis,* 18-20 parts of *Rhizoma Atractylodis,* 10-12 parts of *Herba Moslae,* 20-24 parts of *Folium Artemisiae Argyi,* 0.4-2.5 parts of *Flos Caryophylli,* 4-8 parts of *Herba Menthae Haplocalycis*, and 0.05-0.15 parts of natural borneol.

Preferably, the above pharmaceutical composition further comprises a pharmaceutical excipient.

Preferably, the pharmaceutical composition further comprises, in parts by weight, 0.5-1.5 parts of a pharmaceutical excipient, wherein the pharmaceutical excipient can be selected from sucralose and/or sodium benzoate.

An antibacterial and antiviral pharmaceutical volatile oil is prepared by a method comprising the following steps:

1) adding water to *Herba Pogostemonis, Rhizoma Atractylodis, Herba Moslae, Folium Artemisiae Argyi*, *Flos Caryophylli*, and *Herba Menthae Haplocalycis* respectively, and distilling for extraction of volatile oil, to give the volatile oil of each of the above raw materials, and then mixing the volatile oil of each of the above raw materials to give a mixed volatile oil; or

mixing *Herba Pogostemonis, Rhizoma Atractylodis, Herba Moslae, Folium Artemisiae Argyi, Flos Caryophylli,* and *Herba Menthae Haplocalycis*, then adding water and distilling for extraction, to give a total volatile oil; or

mixing and placing *Herba Pogostemonis, Rhizoma Atractylodis, Herba Moslae, Folium Artemisiae Argyi, Flos Caryophylli*, and *Herba Menthae Haplocalycis* in a supercritical carbon dioxide extraction device, and extracting volatile oil with supercritical extraction by introducing liquid carbon dioxide, to give the total volatile oil; and

2) adding natural borneol to the mixed volatile oil or total volatile oil, heating to dissolve the natural borneol, to give the pharmaceutical volatile oil, or

adding the natural borneol to a solvent at 50-60°C and stirring to dissolve, cooling down to room temperature, then adding to the mixed volatile oil or total volatile oil, to give the pharmaceutical volatile oil.

Preferably, heating temperature after addition of the natural borneol is not above 60 °C. Preferably, the preparation methods for the volatile oil of each of the raw materials are as follows:

*Herba Pogostemonis* is added with 5-8 times its own weight in water, and soaked overnight, followed by extracting by steam distillation for 4-8h;

*Rhizoma Atractylodis* is added with 5-8 times its own weight in water and soaked for 20-60 min, followed by extracting by steam distillation for 4-6h;

*Herba Moslae* is added with 5-8 times its own weight in water and soaked for 1-3h, followed by extracting by steam distillation for 2-4h;

*Folium Attemisiae Argyi* is added with 6-10 times its own weight in water and soaked for 4-8h, followed by extracting by steam distillation for 4-6h;

*Flos Caryophylli* is added with 5-8 times its own weight in water and soaked overnight, followed by extracting by steam distillation for 4-8h;

*Herba Menthae Haplocalycis* is added with 5-8 times its own weight in water and soaked for 1-3 h, followed by extracting by steam distillation for 4-6h.

A preparation method for the total volatile oil is as follows:

*Herba Pogostemonis, Rhizoma Atractylodis, Herba Moslae, Folium Artemisiae Argyi, Flos Caryophylli*, and *Herba Menthae Haplocalycis* are mixed and added with 5-10 times their own weight in water, and soaked for 1-8h, followed by extracting by steam distillation for 2-8h; or

*Herba Pogostemonis, Rhizoma Atractylodis, Herba Moslae, Folium Artemisiae Argyi, Flos Caryophylli*, and *Herba Menthae Haplocalycis* are mixed and placed in a supercritical carbon dioxide extraction device, followed by extraction of the volatile oil by introducing liquid carbon dioxide, with an extraction pressure of 20-40 Mpa, an extraction temperature of 40-70 °C, a separation temperature of 30-60 °C and extraction time of 0.5-10h.

Antibacterial and antiviral pharmaceutical aromatic water is provided, and *Herba Pogostemonis, Rhizoma Atractylodis, Herba Moslae, Folium Artemisiae Argyi, Flos Caryophylli*, and *Herba Menthae Haplocalycis* are mixed and added with 5-12 times their own weight in water, soaked overnight and then distilled, to give the pharmaceutical aromatic water.

Provided is use of the pharmaceutical composition or the pharmaceutical volatile oil or the pharmaceutical aromatic water in preparing a medicament for anti-bacteria, anti-virus, clearing heat, reducing fever and/or treating a respiratory disease.

The pharmaceutical composition, pharmaceutical volatile oil, pharmaceutical aromatic water of the present application, or the medicament prepared therefrom have a good therapeutic effect on fever and a respiratory system disease, such as rhinitis, pharyngitis, pneumonia, cough and asthma, and can effectively resist bacteria and virus, and relieve various symptoms caused by bacterial and/or viral infection.

Further, said virus includes SARS-CoV-2.

Further, rhinitis in the traditional Chinese medicine is generally classified as four types, including three deficiency syndromes and one excess syndrome. Among them, the three deficiency syndromes include: 1) deficiency of lung qi: lung opens at nose, and lung deficiency naturally causes nasal obstruction, and further some nasal symptoms; 2) deficiency of spleen qi: spleen is the mother of lung, and spleen deficiency also causes lung deficiency, which will also result in nasal symptoms, including nose itching, clear nasal discharge, persistent clear nasal discharge, sneezing, and persistent sneezing; 3) kidney yang insufficiency and kidney qi deficiency: kidney and lung are connected with each other, and when the kidney is weak, it also causes the nasal symptoms. The above three deficiency syndromes are actually associated with lung. In addition, the excess syndrome in the syndrome typing relates to hide heat in lung meridian, and belongs to heat pattern/syndrome, and also is caused by lung heat itself. The pharmaceutical composition of the present application can effectively treat the above four types of rhinitis.

Preferably, the above medicament can be prepared into multiple dosage forms, such as spray, distillate medicinal water, aerosol, dropping pills, soft capsules, nasal drops or preparations for atomization inhalation.

Preferably, a preparation method for the spray is as follows:

the pharmaceutical volatile oil is diluted with a diluent, stirred well, and filtrated to give the spray; or natural borneol and the pharmaceutical excipient are added to a solvent at 50-60 °C, stirred to dissolve, and cooled down to room temperature, and then the mixed volatile oil or the total volatile oil is added thereto, diluted with a diluent, stirred well and filtrated to give the spray;
a preparation method for the distillate medicinal water is as follows:
the pharmaceutical excipient is added to the pharmaceutical aromatic water and stirred well, to give the distillate medicinal water;
a preparation method for the aerosol is as follows:

the pharmaceutical volatile oil is diluted with a diluent, stirred well, filtrated, filled in a pressure container, capped and filled with a propellant to give the aerosol; or
natural borneol and the pharmaceutical excipient are added to a solvent at 50-60 °C, stirred to dissolve, and cooled down to room temperature, and then the mixed volatile oil or the total volatile oil is added thereto, diluted with a diluent, stirred well, filtrated, filled in a pressure container, capped and filled with a propellant, to give the aerosol;

a preparation method for the dropping pill is as follows:
the pharmaceutical volatile oil is prepared into volatile oil microcapsule powder by microencapsulation technology, and mixed with a dropping pill matrix, to give the dropping pill;
a preparation method for the soft capsule is as follows:
the pharmaceutical volatile oil is encapsulated with a soft capsule material to give the soft capsule;
a preparation method for the nasal drop is as follows:
the pharmaceutical volatile oil is dissolved in an anhydrous ethanol solution of a gel matrix material, added with a diluent, stirred well, and dried under reduced pressure to remove anhydrous ethanol, to give the oleogel nasal drop;
a preparation method for the preparations for atomization inhalation is as follows:
the pharmaceutical volatile oil is diluted with a diluent, stirred well, filtrated and filled.

Further preferably, a preparation method for the dropping pill is as follows:

A. an emulsifier, corn protein, and sunflower phospholipid are mixed in the pharmaceutical volatile oil, wherein the mass fractions thereof in the pharmaceutical volatile oil are 0.5-1.5%, 0.5-2%, and 0.1-2% respectively;

B. starch sodium octenyl succinate is dissolved in water, to prepare an aqueous solution with a mass fraction of 15-50%;

C. the two solutions prepared in A and B are mixed in a weight ratio of 1: 1-2, emulsified with stirring by using a 10000-14000r/min ultra-high-speed stirrer, and homogenized 2-3 times using a high pressure homogenizer;

D. the homogenized solution obtained in C is spray-dried to give a volatile oil microcapsule powder; and

E. the volatile oil microcapsule powder and a dropping pill matrix are prepared into a dropping pill in a mass ratio of 1:2, wherein the dropping pill matrix material is polyethylene glycol 4000 : polyethylene glycol 6000 (1:1).

Preferably, the solvent is any one of medium chain triglycerides, 1-98% ethanol, anhydrous ethanol, propylene glycol, glycerol or edible oils;

the diluent is any one of medium chain triglycerides, 1-98% ethanol, anhydrous ethanol, propylene glycol, glycerol or edible oils;

the propellant is nitrogen gas, carbon dioxide, heptafluoropropane (HFC-227ea), tetrafluoroethane (HFC-134a), 1,3,3,3-tetrafluoropropene (HFO-1234ze), 2,3,3,3-tetrafluoropropene (HFO-1234yf) or compressed air.

Preferably, a ratio of liquid phase and gas phase in the aerosol can be selected from any one of 2:8, 3:7, 4:6, 5:5, and 6:4, and a pressure in the pressure container is 0.6-1MPa.

Further preferably, the edible oil can be selected from semen cannabis oil, sacha inchi oil, olive oil, coconut oil, pomegranate seed oil, wheat germ oil, walnut oil, avocado oil, linseed oil, grape seed oil, tea seed oil, perilla oil, pumpkin seed oil, sunflower seed oil, canola seed oil, cranberry seed oil, astaxanthin oil, deep-sea fish oil, soybean oil, peanut oil, sesame oil, camellia oil, corn oil, prinsepia utilis royle oil, borage oil, evening primrose oil, palm oil, and the like.

Preferably, the gel matrix material can be selected from ethyl cellulose and the like.

Further, the preparation methods for the spray, distillate medicinal water, aerosol, dropping pill, soft capsule, nasal drop, preparation for atomization inhalation, and the like according to the present application are not limited to the above methods.

In summary, the pharmaceutical composition of the present application can be used for preparing a medicament for anti-bacteria, anti-virus, clearing heat, reducing fever and/or treating a respiratory disease, and has good therapeutic effects on fever and a respiratory system disease, such as rhinitis, pharyngitis, pneumonia, cough and asthma, and has significant efficacy on a disease caused by bacterial and/or viral infection. It has good application potential in the treatment of mild type of COVID-19 (with mild clinical symptoms, no manifestation of pneumonia on imaging, and positive nucleic acid test) and ordinary type of COVID-19 (with fever and respiratory symptoms, visible pneumonia manifestation on imaging, viral pneumonia manifestations, and positive nucleic acid test).

**Description of the drawings**

FIG. 1 shows the toxic effect of the spray in Example 4 on Vero E6 cells, $CC_{50} > 1/100$ (v/v);

FIG. 2 shows the toxic effect of Remdesivir on Vero E6 cells, $CC_{50} > 200 \mu M$;

FIG. 3 shows the inhibitory effect of the spray in Example 4 on cell death induced by SARS-COV-2 (simultaneous addition of the drug and virus), $IC_{50} > 1/100$ (v/v);

FIG. 4 shows the inhibitory effect of Remdesivir on cell death induced by SARS-COV-2 (simultaneous addition of the drug and virus), $IC_{50} = 1.118 \pm 0.052 \mu M$);

FIG. 5 shows the inhibitory effect of the spray in Example 4 on cell death induced by SARS-COV-2 (addition of the drug after infection), $IC_{50} > 1/100$ (v/v);

FIG. 6 shows the inhibitory effect of Remdesivir on cell death induced by SARS-COV-2 (addition of the drug after

infection), $IC_{50} = 0.825 \pm 0.128\ \mu M$;

FIG. 7 shows the killing effect of the 1/100 (v/v) spray on SARS-CoV-2;

FIG. 8 shows the killing effect of $H_2O_2$ (0.01%) on SARS-CoV-2.

## Detailed Description of the Invention

The technical solutions in the Examples of the present application will be clearly and completely described below, and the general principles as defined herein may be implemented in other examples without departing from the spirit or scope of the present application. Therefore, the present application will not be limited to these examples shown herein, and should conform to the broadest scope that is consistent with the principle and novel features disclosed herein.

In the examples, Asian mouse-lung-adaptation strain of influenza A virus A/FM/1/34 (H1N1) (FM1) was provided by Institute of Virology, Chinese Academy of Preventive Medicine, stored in liquid nitrogen in our laboratory; A/PR/8/34 (H1N1) and A/Aichi/2/1968 (H3N2) were purchased from the American Type Culture Collection (ATCC); 2009 H1N1 influenza A virus strain (A/Guangzhou/GIRD07/09, H1N1, Genbank No. HM014332.1), influenza B virus (B/Guangzhou/GIRD08/09, FluB) were the clinical isolated strains from Department of Virology, State Key Laboratory of Respiratory Diseases, Guangzhou Medical University; H9N2 influenza A virus (A/Ahicken/Guangdong/1996, H9N2), H6N2 influenza A virus (A/Duck/Guangdong/2009, H6N2), and H7N3 influenza A virus (A/Duck/Guangdong/1994, H7N3) were donated from College of Veterinary Medicine, South China Agricultural University. *In vitro* anti-SARS-CoV-2 activity tests were entrusted to Kunming Institute of Zoology, Chinese Academy of Sciences.

## Example 1

For an antibacterial and antiviral pharmaceutical composition, the raw materials comprise: 885g of *Herba Pogostemonis,* 1869g of *Rhizoma Atractylodis,* 1064g of *Herba Moslae,* 2174g of *Folium Artemisiae Argyi,* 46g of *Flos Caryophylli,* 532g of *Herba Menthae Haplocalycis* and 10g of natural borneol.

The above pharmaceutical composition was used for preparing the pharmaceutical volatile oil, and the preparation method was as follows:

*Herba Pogostemonis,* in a form of herbal decoction pieces, was crushed and sieved by a 20 mesh sieve, soaked overnight with 6 times its own weight in water, and then extracted by steam distillation for 6h; and the obtained *Herba Pogostemonis* volatile oil was collected, with the extraction rate of 2.26 %.

Coarse powder of the raw *Rhizoma Atractylodis* was taken, soaked for 0.5h with 6 times its own weight in water, and then extracted by steam distillation for 5h, and the obtained *Rhizoma Atractylodis* volatile oil was collected, with the extraction rate of 1.07%.

*Herba Moslae* was taken, soaked for 2h with 6 times its own weight in water, and then extracted by steam distillation for 3h, and the obtained *Herba Moslae* volatile oil was collected, with the extraction rate of 0.94%.

*Folium Artemisiae Argyi* was taken and coarsely crushed, soaked for 6h with 8 times its own weight in water, and then extracted by steam distillation for 5h, and the obtained *Folium Artemisiae Argyi* volatile oil was collected, with the extraction rate of 0.46%.

*Flos Caryophylli,* in the form of herbal decoction pieces, was soaked overnight with 6 times its own weight in water, and then extracted by steam distillation for 6h, and the obtained *Flos Caryophylli* volatile oil was collected, with the extracting rate of 10.88%.

*Herba Menthae Haplocalycis* in the form of herbal decoction pieces was soaked for 2h with 6 times its own weight in water, and extracted by steam distillation for 5h, and the obtained *Herba Menthae Haplocalycis volatile* oil was collected, with the extraction rate of 0.84%.

The extracted *Herba Pogostemonis* volatile oil, *Rhizoma Atractylodis* volatile oil, *Herba Moslae* volatile oil, *Folium Artemisiae Argyi* volatile oil, *Flos Caryophylli* volatile oil, and *Herba Menthae Haplocalycis* volatile oil were mixed, added with natural borneol, and properly heated (not above 60 °C) to completely dissolve the natural borneol, to give the pharmaceutical volatile oil.

## Example 2

For an antibacterial and antiviral pharmaceutical composition, the raw materials comprise: 663.5g of *Herba Pogostemonis,* 1402g of *Rhizoma Atractylodis,* 798g of *Herba Moslae,* 1630.5g of *Folium Artemisiae Argyi,* 32g of *Flos Caryophylli*, 106.5g of *Herba Menthae Haplocalycis* and 2.5g of natural borneol.

The preparation method for preparing the above pharmaceutical composition into the pharmaceutical volatile oil was as follows:

Each of the raw materials was crushed into coarse powder, mixed together and placed in a HA220-40-48 supercritical carbon dioxide extraction device, followed by extraction of the volatile oil by introducing liquid carbon dioxide, with the extraction pressure of 28-30 Mpa, the extraction temperature of 55 °C, the separation temperature of 45°C and the extraction time of 3h; 106.5g of the extract (the total volatile oil) was obtained, then added with the natural borneol, and properly heated (not above 60 °C) to completely dissolve the natural borneol, to give the pharmaceutical volatile oil.

**Example 3**

The efficacy research tests were carried out on the pharmaceutical volatile oil of Example 1:
The doses for mice in the efficacy research were 65 mg/kg, 130 mg/kg and 260 mg/kg, respectively.
The doses for guinea pigs in the efficacy research were 32 mg/kg, 64 mg/kg and 128 mg/kg, respectively.
The doses for rats in the efficacy research were 37 mg/kg, 74 mg/kg and 148 mg/kg, respectively.

1. Concentrated ammonia water induced cough tests in mice (relieving cough effects)

1.1 Experimental Method

ICR mice were used, half male and half female. The mice were randomly divided into 6 groups according to weight levels: normal control group, model control group, pentoxyverine citrate 20 mg/kg group, and a low dose group (65 mg/kg), a medium dose group (130 mg/kg) and a high dose group (260 mg/kg) of the pharmaceutical volatile oil, 14 mice for each group. The mice were intragastrically administrated with 20 mL/kg (formulated with soybean oil to the corresponding concentrations) for 7d, once per day. The mice in the normal control and model control groups were intragastrically administrated with an equal amount of soybean oil.

1.2 Determination of indicators

At 1 hour after the last administration, the mice were put in a 500 mL transparent sealed container, and the atomized concentrated ammonia water was uniformly sprayed into the container using an ultrasonic atomizer, continuously for 20s. The phenomenon of shrinking abdomen and opening mouth in mice was taken as cough, and cough incubation period and cough frequency of mice within 2 min after being put in the container were recorded.

1.3 Experimental Results

Compared with the model control group, the cough incubation period of the mice was significantly prolonged, and the cough frequency of the mice was significantly reduced ($P < 0.01$) in each dose group of the pharmaceutical volatile oil. The results were shown in Table 1.

Table 1 Effect of the pharmaceutical volatile oil on concentrated ammonia water induced cough in mice ($\bar{x}\pm$s, n=14)

| Groups | Dose (mg/kg) | Cough incubation period (s) | Frequency of cough (time/2 min) |
|---|---|---|---|
| normal control | - | 0±0 | 0±0 |
| model control | - | 11.8±3.2 | 126.7±22.8 |
| pentoxyverine citrate | 20 | 26.3±3.3** | 55.8±14.7** |
| | 65 | 18.7±1.8** | 90.0±10.2** |
| pharmaceutical volatile oil | 130 | 19.3±2.2** | 100.5±11.2** |
| | 260 | 21.3±2.0** | 77.5±17.2** |

Note: compared with the model control group, ** $P < 0.01$.

Under the experimental conditions and the designed dosages in this experiment, the pharmaceutical volatile oil has an obvious effect of relieving cough.

2. Effect on asthmatic guinea pigs induced by acetylcholine combined with histamine phosphate (antiasthmatic effect)

2.1 Experimental Method

Conventional guinea pigs were used, half male and half female. The guinea pigs were put into a glass cover (4L volume) one by one, and a mixed solution of equal volumes of 0.1% histamine phosphate and 2% acetylcholine chloride was sprayed into the glass cover using an ultrasonic atomizer for 15s; and pre-administration asthma incubation period of guinea pigs (time from spray to convulsion) was recorded one by one. The guinea pigs with the asthma incubation period of not more than 120s were considered as qualified guinea pigs, while those beyond the asthma incubation period were considered as not sensitive, and were removed.

The selected qualified guinea pigs were randomly divided into 5 groups according to the pre-administration asthma incubation period: model control group, aminophylline 60 mg/kg group, and a low dose group (32 mg/kg), a medium dose group (64 mg/kg) and a high dose group (128 mg/kg) of the pharmaceutical volatile oil, 12 guinea pigs for each group. The guinea pigs were intragastrically administrated with 4 mL/kg (formulated with soybean oil to the corresponding concentrations) for 7d, once a day. The guinea pigs in the model control group were intragastrically administrated with an equal amount of soybean oil.

2.2 Determination of indicators

At 1 hour after the last administration, the guinea pigs were recorded for the post-administration asthma incubation period using the same method. If the guinea pigs did not show asthma symptoms within 3 min, they were then calculated as 180s.

2.3 Experimental Results

Compared with the model control group, the post-administration asthma incubation period and the extension value of the post-administration asthma incubation period (post-administration incubation period - pre-administration incubation period) of the guinea pigs in each dose group of the pharmaceutical volatile oil were significantly increased $(P < 0.01)$. The results were shown in Table 2.

Table 2 Effect of the pharmaceutical volatile oil on asthma incubation period of guinea pigs induced by acetylcholine and histamine phosphate ($\bar{x} \pm s$, n=12)

| Groups | Dose (mg/kg) | cough incubation period (s) | | |
| --- | --- | --- | --- | --- |
| | | Pre-administration | Post-administration | extension value (post - pre) |
| model control | - | 32.5±8.1 | 30.2±4.1 | -2.3±9.3 |
| aminophylline | 60 | 33.1±9.2 | 123.4±50.3** | 90.3±51.1** |
| pharmaceutical volatile oil | 32 | 32.6±4.6 | 86.4±46.5** | 53.8±45.8** |
| | 64 | 33.1±7.5 | 88.3±51.1** | 55.2±51.2** |
| | 128 | 32.7±7.3 | 96.0±53.4** | 63.3±50.3** |

Note: compared with the model control group, ** $P$< 0.01.

Under the experiment conditions and the designed dosages in this experiment, the pharmaceutical volatile oil has an obvious antiasthmatic effect.

3. Effect on tracheal excretion induced by intraperitoneal injection of phenol red in mice (phlegm-reducing effect)

3.1 Experimental Method

ICR mice, half male and half female, were randomly divided into 5 groups according to weight levels: model control group, ambroxol hydrochloride 30 mg/kg group, and a low dose group (65 mg/kg), a medium dose group (130 mg/kg) and a high dose group (260 mg/kg) of the pharmaceutical volatile oil, 14 mice for each group. The mice were intragastrically administrated with 20 mL/kg (formulated with soybean oil to the corresponding concentrations) for 7 days, once a day. The mice in the model control group were intragastrically administrated with an equal amount of soybean oil.

3.2 Determination of indicators

At 30 min after the last administration, the mice were intraperitoneally injected with 20 mL/kg of 5% phenol red physiological saline solution, and executed after 30 min; the trachea was exposed, the peritracheal tissue tissues were peeled, a section of the trachea from the subthyroid cartilage to bronchus branches was cut and soaked in a test tube filled with 2 mL of 5% $NaHCO_3$ solution, shaken for washing off the excreted phenol red completely, and allowed to leave overnight, to give a transparent supernatant, which was subjected to determination of the absorbance value of the trachea eluate at 546 nm.

3.3 Experimental Results

Compared with the model control group, the tracheal excretion amount of phenol red of the mice in each dose group of the pharmaceutical volatile oil was significantly increased (P< 0.01). The results were shown in Table 3.

Table 3 Effect of the pharmaceutical volatile oil on tracheal excretion of phenol red of mice ($\overline{x}\pm$s, n=14)

| Groups | Dose (mg/kg) | Tracheal excretion amount of phenol red (OD value) |
|---|---|---|
| model control | - | 0.202±0.043 |
| ambroxol hydrochloride | 30 | 0.566±0.201** |
| pharmaceutical volatile oil | 65 | 0.301±0.116** |
| | 130 | 0.388±0.152** |
| | 260 | 0.372±0.127** |

Note: compared with the model control group, ** $P$ < 0.01.

Under the experimental conditions and the designed dosages in this experiment, the pharmaceutical volatile oil has an obvious phlegm-reducing effect.

4. Effect on capillary discharge sputum volume in rats (phlegm-reducing effect)

4.1 Experimental Method

SD rats, half male and half female, were randomly divided into 5 groups according to weight levels: model control group, carbocisteine 200 mg/kg group, and a low dose group (37 mg/kg), a medium dose group (74 mg/kg) and a high dose group (148 mg/kg) of the pharmaceutical volatile oil, 12 rats for each group. The rats were intragastrically administrated with 10 mL/kg (formulated with soybean oil to the corresponding concentrations) for 7 days, once a day. The rats in the model control group were intragastrically administrated with an equal amount of soybean oil.

4.2 Determination of indicators

Rats were anesthetized and fixed in supine position at 40 min after the last administration, and the rats were cut the skin of the middle of neck, to separate the trachea; a glass capillary was inserted through a small hole pricked by a sharp injection needle between the two cricoid cartilages in the middle of the lower edge of the thyroid cartilage, to allow the capillary to just contact the bottom surface of the trachea; the sputum in the trachea was absorbed into the capillary; when the capillary was filled with sputum, it is immediately replaced with another capillary having the same diameter; after continuous collection for 2h, the liquid column in the capillary was measured in length with a graduated scale, to evaluate the sputum excretion volume.

4.3 Experimental Results

Compared with the model control group, the sputum excretion volume of the rats in each dose group of the pharmaceutical volatile oil was significantly increased (P< 0.05 or $P$< 0.01). The results were shown in Table 4.

Table 4 Effect of the pharmaceutical volatile oil on capillary discharge sputum volume in rats ($\overline{x}\pm$s, n=12)

| Groups | Dose (mg/kg) | Sputum excretion volume (cm) |
|---|---|---|
| model control | - | 7.40±1.39 |
| carbocisteine | 200 | 18.11±4.32** |
| pharmaceutical volatile oil | 37 | 10.11±3.67* |
| | 74 | 11.32±2.81** |

(continued)

| Groups | Dose (mg/kg) | Sputum excretion volume (cm) |
|---|---|---|
| | 148 | 12.96±4.01 ** |
| Note: compared with the model control group, * $P< 0.05$, ** $P < 0.01$. | | |

Under the experimental conditions and the designed dosages in this experiment, the pharmaceutical volatile oil has an obvious phlegm-reducing effect.

5. Effect on lipopolysaccharide (LPS) induced rat fever model (fever-relieving effect)

5.1 Experimental Method

SD rats were used, half male and half female. The SD rats were subjected to adaptive rectal temperature measurement 2 days earlier, twice per day, and the average value thereof was used as the basal body temperature. A rat with a single body temperature of more than 38 °C or with a temperature difference of more than 0.5 °C between two measurements were removed. The qualified rats were randomly divided into 6 groups according to body temperature levels: normal control group, model control group, aspirin 200 mg/kg group, and a low dose group (37 mg/kg), a medium dose group (74 mg/kg) and a high dose group (148 mg/kg) of the pharmaceutical volatile oil, 12 rats for each group. The rats were intragastrically administrated with 10 mL/kg (formulated with soybean oil to the corresponding concentrations) for 7 days, once a day. The rats in the normal control and model control groups were intragastrically administrated with an equal amount of soybean oil.

5.2 Determination of indicators

Rats were fasted but allowed to water 12 hours before the experiment; the basal body temperatures of the rats were measured on the experiment day; at 1 hour after the last administration, the rats in the normal control group were intraperitoneally injected with sterile physiological saline, the rats in the other groups were intraperitoneally injected with LPS (20 μg/kg) for modeling; after modeling, the states and body temperature changes of the rats were recorded for 7h, and the body temperature was measured every 1 hour. The difference ($\Delta T$) of the body temperature every 1 hour and the basal body temperature was used as the indicators.

5.3 Experimental Results

The rats in the normal control group had no obvious change of the body temperature in 7h, while the rats in the model control group had a gradually increased body temperature after LPS injection; and it had a significant difference compared with the model control group ($P< 0.01$), and it reached its peak value at 6h. Compared with the model control group, the body temperature in each dose group of the pharmaceutical volatile oil was significantly reduced at 2h-7h ($P< 0.05$ or $P< 0.01$). The results were shown in Table 5.

Table 5 Effect of the pharmaceutical volatile oil on LPS-induced fever in rats ($\bar{x}\pm$s, n=12)

| Groups | Dose (mg/kg) | Temperature difference ($\Delta$T) °C | | | | | | |
|---|---|---|---|---|---|---|---|---|
| | | 1h | 2h | 3h | 4h | 5h | 6h | 7h |
| normal control | - | -0.03±0.39 | -0.27±0.39 | -0.16±0.42 | -0.17±0.25 | -0.03±0.31 | -0.04±0.33 | -0.04±0.23 |
| model control | - | 0.52±0.22## | 0.93±0.39## | 1.36±0.33## | 1.50±0.55## | 1.91±0.28## | 2.34±0.20## | 1.75±0.24## |
| aspirin | 200 | 0.10±0.34** | 0.43±0.29** | 0.42±0.24** | 0.46±0.34** | 0.79±0.17** | 0.88±0.18** | 0.53±0.24** |
| pharmaceutical volatile oil | 37 | 0.32±0.19 | 0.61±0.27* | 0.96±0.27* | 1.17±0.23* | 1.37±0.39** | 1.97±0.20** | 1.43±0.37 |
| | 74 | 0.29±0.40 | 0.51±0.33** | 0.82±0.49** | 1.08±0.41* | 1.41±0.23** | 1.74±0.15** | 1.43±0.11* |
| | 148 | 0.30±0.27 | 0.43±0.27** | 0.52±0.31** | 0.72±0.26** | 0.97±0.23** | 1.11±0.20** | 0.87±0.30** |

Note: compared with the normal control group, ## $P$ < 0.01; compared with the model control group, * $P$ < 0.05, ** $P$ < 0.01.

Under the experimental conditions and the designed dosages in this experiment, the pharmaceutical volatile oil has an obvious fever-relieving effect.

6. Effect on acute pharyngitis model in rat

6.1 Experimental Method

Wistar rats were used, half female and half male, and the rats were grasped and fixed by hand; the lower jaws of the rats were opened using a tweezer and fixed; the spray head of a spray extended from the mouth to the throat in the rats, and sprayed 25% ammonia water to the throat of the rats once (about 0.2 mL); and the spray was continued for 3 days, twice a day, to induce the formation of acute inflammation of the pharyngeal mucosa in the rats due to irritation. After continuous molding for 3 days, the rats were randomly divided into model control group, dexamethasone 5 mg/kg group, and a low dose group (37 mg/kg), a medium dose group (74 mg/kg) and a high dose group (148 mg/kg) of the pharmaceutical volatile oil, 12 rats for each group. Another 12 rats from the same batch were used as normal control group. The rats were intragastrically administrated with 10 mL/kg (formulated with soybean oil to the corresponding concentrations) for 3 days, once a day. The rats in the normal control and model control groups were intragastrically administrated with an equal amount of soybean oil.

6.2 Determination of indicators

Rats were anesthetized at 1 hour after the last administration, subjected to abdominal aorta blood sampling and then executed; serum was separated, to determine the levels of IL-6 and TNF-$\alpha$ therein by ELISA method.

6.3 Experimental Results

Compared with the normal control group, the content of IL-6 and TNF-$\alpha$ in serum of the rats in the model control group was significantly increased *(P < 0.01)*. Compared with the model control group, each of the dose groups of the pharmaceutical volatile oil could significantly reduce the IL-6 and TNF-$\alpha$ content in serum of the rats *(P < 0.05 or P < 0.01)*. The results were shown in Table 6.

Table 6 Effect of the pharmaceutical volatile oil on acute pharyngitis rats ($\overline{x}\pm$s, n=12)

| Groups | Dose (mg/kg) | IL-6 (pg/mL) | TNF-$\alpha$ (pg/mL) |
|---|---|---|---|
| normal control | - | 135.1$\pm$10.5 | 154.8$\pm$12.5 |
| model control | - | 233.5$\pm$9.1## | 238.9$\pm$8.9## |
| dexamethasone | 5 | 146.3$\pm$10.4** | 218.2$\pm$5.8** |
| pharmaceutical volatile oil | 37 | 190.2$\pm$7.9** | 231.8$\pm$8.2** |
| | 74 | 183.8$\pm$9.5** | 223.3$\pm$6.8** |
| | 148 | 171.5$\pm$11.0** | 217.7$\pm$8.0** |

Note: compared with the normal control group, ##*P* < 0.01; compared with the model control group, **P* < 0.05, ***P* < 0.01.

Under the experimental conditions and the designed dosages in this experiment, the pharmaceutical volatile oil could obviously inhibit inflammation reaction of acute pharyngitis model rats.

7. Protection effect on virus-induced death in mice

7.1 Experimental Method

Asian mouse-lung-adaptation strain of influenza A virus A/FM/1/34 (H1N1) (FM1) was inoculated in a 9-day-old chick embryo allantoic cavity before using, and conventionally cultured; allantoic fluid was collected freshly and aseptically after 48h, and subcultured twice, to measure the median lethal dose (LD$_{50}$) in ICR mice. ICR mice, half male and half female, were randomly divided into 6 groups according to weight levels: normal control group, model control group, ribavirin 100 mg/kg group, and a low dose group (65 mg/kg), a medium dose group (130 mg/kg) and a high dose group (260 mg/kg) of the pharmaceutical volatile oil, 16 mice for each group. The mice were lightly anaesthetized with diethyl ether, and each of the mice was inoculated intranasally with 25 $\mu$L of virus fluid, with the infection dose of 25 times LD$_{50}$; while the mice in the normal control group were dropped intranasally with an equal amount of sterile physiological saline. The mice in each dose group of the pharmaceutical volatile oil were intragastrically administrated with 20 mL/kg (formulated with soybean oil to the corresponding concentrations), and the mice in the ribavirin group were administrated by intraperitoneal injection for 7 days, once a day. The mice in the normal control and model control groups were intragastrically administrated with an equal amount of soybean oil.

7.2 Determination of indicators

The disease symptoms of mice were observed daily, and death time, death number and weight were recorded, for 14 days. The mouse that was alive on day 14 was calculated based on 14 days, and the mouse that died within 24 hours post infection was regarded as non-infection death.

7.3 Experimental Results

The mice in the normal control group were in good spirits and agility, and had smooth and shiny fur, normal breathing frequency, normal food and water intake and increased weight daily. The mice infected with influenza virus showed head-twitches, rapid breathing rate, mainly abdominal breathing, slowing in action, curling, shrugged hair, and significant decrease of the food and water intake amount, at 24 hours post infection. Starting from day 4, the mice began to death (reaching a peak on day 7), and the states of the mice in each treatment group were obviously better than those in the model group.

There is no mouse died in the normal control group, while the survival rate of the mice in the model control group was only 6.2%; compared with the model control group, the survival rates of the low, medium and high dose groups of the pharmaceutical volatile oil were 25.0%, 37.5%, and 56.2% respectively, which were significantly increased ($P< 0.05$ or $P< 0.01$). The results were shown in Table 7.

Table 7 Effect of the pharmaceutical volatile oil on survival rate of influenza virus FM1 infected mice (n=16)

| Groups | Dose (mg/kg) | Survival number | Survival rate (%) |
| --- | --- | --- | --- |
| normal control | - | 16 | 100 |
| model control | - | 1 | 6.2## |
| ribavirin | 100 | 13 | 81.2** |
| | 65 | 4 | 25.0' |
| pharmaceutical volatile oil | 130 | 6 | 37.5* |
| | 260 | 9 | 56.2** |

Note: compared with the normal control group, ## $P< 0.01$; compared with the model control group, * $P< 0.05$, "$P< 0.01$.

Under the experiment conditions and the designed dosages in this experiment, the pharmaceutical volatile oil could obviously improve the survival rate of influenza virus FM1 infected mice, and has an obvious anti-viral effect.

8. Effect on viral pneumonia of mice infected with influenza virus

8.1 Experimental Method

Asian mouse-lung-adaptation strain of influenza A virus A/FM/1/34 (H1N1) (FM1) was inoculated in a 9-day-old chick embryo allantoic cavity before using, and conventionally cultured; allantoic fluid was collected freshly and aseptically after 48h, and subcultured twice, to measure the median lethal dose ($LD_{50}$) in ICR mice. ICR mice, half male and half female, were randomly divided into 6 groups according to weight levels: normal control group, model control group, oseltamivir 27.5 mg/kg group, and a low dose group (65 mg/kg), a medium dose group (130 mg/kg) and a high dose group (260 mg/kg) of the pharmaceutical volatile oil, 14 mice for each group. The mice were lightly anaesthetized with diethyl ether, and each of the mice was inoculated intranasally with 20 $\mu$L of virus fluid, with the infection dose of 25 times $LD_{50}$; while the mice in the normal control group were dropped intranasally with an equal amount of sterile physiological saline. The mice in each dose group of the pharmaceutical volatile oil were intragastrically administrated with 20 mL/kg (formulated with soybean oil to the corresponding concentrations) for 7 days, once a day. The mice in the normal control and model control groups were intragastrically administrated with an equal amount of soybean oil.

8.2 Determination of indicators

At 1 hour after the last administration, the mice were weighed, executed and dissected to remove the whole lung, and the whole lung was weighed to calculate lung index value and lung index inhibition rate. The lung tissue was prepared with physiological saline into 10% lung tissue homogenate; the prepared lung tissue homogenate was subjected to cryogenic centrifugation, and the content of IL-8 and TNF-$\alpha$ in the lung tissue was determined by ELISA method.

$$\text{lung index} = [\text{lung weight (g)} / \text{body weight (g)}] \times 100;$$

$$\text{lung index inhibition rate} = (\text{mean lung index of model control group} - \text{mean lung index of test group}) / \text{mean lung index of model control group.}$$

8.3 Experimental Results

Compared with the normal control group, the lung index of the mice in the model control group was significantly increased $(P < 0.01)$; and compared with the model control group, the lung index of each of the dose groups of the pharmaceutical volatile oil was significantly reduced $(P < 0.05$ or $P < 0.01)$. The results were shown in Table 8.

Table 8 Effect of the pharmaceutical volatile oil on lung index of pneumonia mice infected with influenza virus FM1 ($\bar{x} \pm$s, n=14)

| Groups | Dose (mg/kg) | Lung index | Lung index inhibition rate % |
|---|---|---|---|
| normal control | - | 0.73±0.05 | - |
| model control | - | 1.61±0.24## | - |
| oseltamivir | 27.5 | 0.95±0.13** | 41.0 |
| pharmaceutical volatile oil | 65 | 1.33±0.15* | 17.4 |
| | 130 | 1.23±0.20** | 23.6 |
| | 260 | 1.10±0.13** | 31.7 |

Note: compared with the normal control group, ## $P < 0.01$; compared with the model control group, * $P < 0.05$, ** $P < 0.01$.

Compared with the normal control group, the content of IL-8 and TNF-$\alpha$ in the mouse lung tissue homogenate of the model control group was significantly increased $(P < 0.01)$; and compared with the model control group, the content of IL-8 and TNF-$\alpha$ in the mouse lung tissue homogenate of each dose group of the pharmaceutical volatile oil was significantly reduced $(P < 0.05$ or $P < 0.01)$. The results were shown in Table 9.

Table 9 Effect of the pharmaceutical volatile oil on IL-8 and TNF-$\alpha$ of pneumonia mice infected with influenza virus FM1 ($\bar{x} \pm$s, n=14)

| Groups | Dose (mg/kg) | IL-8 (pg/mL) | TNF-$\alpha$ (pg/mL) |
|---|---|---|---|
| normal control | - | 51.5±10.3 | 35.4±13.4 |
| model control | - | 170.3±35.5## | 113.9±27.1## |
| oseltamivir | 27.5 | 85.3±17.4** | 66.8±19.5** |
| pharmaceutical volatile oil | 65 | 145.5±25.3* | 94.5±21.1* |
| | 130 | 121.3±19.2** | 86.4±22.5* |
| | 260 | 105.5±21.1** | 79.7±15.5** |

Note: compared with the normal control group, ## $P < 0.01$; compared with the model control group, * $P < 0.05$, " $P < 0.01$.

Under the experimental conditions and the designed dosages in this experiment, the pharmaceutical volatile oil could obviously improve viral pneumonia induced by H1N1 influenza.

9. Research on *in vitro anti*-viral effect

9.1 Experimental Method

The cell line selected is canine kidney cells (MDCK), and 7 virus strains are A/PR/8/34 (H1N1), A/Aichi/2/1968 (H3N2), 2009 H1N1 influenza A virus strain (A/Guangzhou/GIRD07/09, H1N1), influenza B virus (B/Guangzhou/GIRD08/09, FluB), H9N2 influenza A virus (A/Ahicken/Guangdong/1996, H9N2), H6N2 influenza A virus (A/Duck/Guangdong/2009, H6N2) and H7N3 influenza A virus (A/Duck/Guangdong/1994, H7N3).

The above 7 influenza virus were amplified in a 9- to 11-day-old chick embryo, and allantoic fluid was collected for determination of hemagglutination titer, stored at -80 °C.

The inhibition of the pharmaceutical volatile oil on influenza virus strains such as H1N1, H3N2, 2009 H1N1 type A, FluB, H9N2, H6N2 and H7N3 was investigated by cytopathic-effect inhibition assay. MDCK cells were conventionally cultured with MEM culture medium containing double antibodies and 10% fetal bovine serum. A 96-well plate covered with a single layer of MDCK cells was allowed to absorb virus for 2h; the test groups were added with the pharmaceutical volatile oil at different concentrations (equal volume); meanwhile, a positive control group (Ribavirin) and a blank control group were set, with 100 $\mu$L/well, and cultured for 2 days at 37 °C, 5% $CO_2$.

9.2 Determination of indicators

The extent of cytopathic effect was recorded as the following 6 grade standard: "-" for normal cell growth without cytopathy; "$\pm$" for cytopathy in less than 10% of the entire cell monolayer; "+" for cytopathy in less than 25% of the entire cell monolayer; "++" for cytopathy in less than 50% of the entire cell monolayer; "+++" for cytopathy in less than 75% of the entire cell monolayer; "++++" for cytopathy in 75% or more of the entire cell monolayer.

Half inhibitory concentration ($IC_{50}$) was calculated as Reed-Muench method, and expressed as selection index SI ($TC_{50}/IC_{50}$), criterion for judgment: SI < 1 represents invalid; SI: 1-2 represents high toxicity and low effect; SI > 2 represents low toxicity and high effect.

9.3 Experimental Results

The non-toxic concentration of the pharmaceutical volatile oil to the MDCK cells was 50 mg/mL, with the half toxic concentration $TC_{50}$ of > 50 mg/mL. The $IC_{50}$ values of the pharmaceutical volatile oil for H1N1, H3N2, 2009 H1N1 type A, FluB, H9N2, H6N2 and H7N3 were 18.4 mg/mL, 36.2 mg/mL, 24.2 mg/mL, 28.5 mg/mL, 48.3 mg/mL, 45.5 mg/mL, and 30.2 mg/mL, respectively. The results were shown in Table 10.

Table 10 *In vitro* anti-influenza virus effect of the pharmaceutical volatile oil

| Virus strains | $TC_{50}$ (concentration, mg/mL) | $IC_{50}$ (concentration, mg/mL) | SI |
|---|---|---|---|
| H1N1 | > 50 | 18.4 | > 2.72 |
| H3N2 | > 50 | 36.2 | > 1.38 |
| 2009 H1N1 type A | > 50 | 24.2 | > 2.07 |
| FluB | >50 | 28.5 | > 1.75 |
| H9N2 | > 50 | 48.3 | > 1.04 |
| H6N2 | > 50 | 45.5 | > 1.10 |
| H7N3 | > 50 | 30.2 | > 1.65 |

Under the experimental conditions and the designed dosages in this experiment, the pharmaceutical volatile oil has obvious inhibitory effects on all 7 influenza virus strains, including H1N1, H3N2, 2009 H1N1 type A, FluB, H9N2, H6N2 and H7N3, and shows stronger effects on H1N1, 2009 H1N1 type A and FluB strains.

10. Protection effect on mice infected with *Diplococcus pneumoniae*

10.1 Experimental Method

ICR mice, half male and half female, were randomly divided into 6 groups according to weight levels: normal control group, model control group, Shuanghuanglian 20 g/kg group, and a low dose group (65 mg/kg), a medium dose group (130 mg/kg) and a high dose group (260 mg/kg) of the pharmaceutical volatile oil, 20 mice for each group. The mice were intragastrically administrated with 20 mL/kg (formulated with soybean oil to the corresponding concentrations) for 7 days, once a day. The mice in the model control group were intragastrically administrated with an equal amount of soybean oil.

10.2 Determination of indicators

At 1 hour after administration on day 4, the mice, except those in the normal control group, were intraperitoneally injected with a solution of *Diplococcus pneumoniae* at 0.5 mL/mouse for 3 days, and the death of mice in each group within 7 days was observed.

10.3 Experimental Results

Compared with the normal control group, the death of the mice infected with *Diplococcus pneumoniae was* significantly increased in 7 days, with the survival rate of 20% *(P< 0.01)*; and compared with the model control group, the mortality of the mice in the medium and high dose groups of the pharmaceutical volatile oil in 7 days was significantly reduced (*P* < 0.05 or *P* < 0.01). The results were shown in Table 11.

Table 11 Effect of the pharmaceutical volatile oil on survival rate of mice infected with *Diplococcus pneumoniae* (n=20)

| Groups | Dose (mg/kg) | Survival number | Survival rate (%) |
|---|---|---|---|
| normal control | - | 20 | 100 |
| model control | - | 4 | 20.0## |
| Shuanghuanglian | 20000 | 12 | 60.0** |
| | 65 | 9 | 45.0 |
| pharmaceutical volatile oil | 130 | 10 | 50.0* |
| | 260 | 12 | 60.0** |

Note: compared with the normal control group, ## $P$ < 0.01; compared with the model control group, * $P$< 0.05, "$P$<0.01.

Under the experimental conditions and the designed dosages in this experiment, the pharmaceutical volatile oil could obviously improve the survival rate of mice infected with *Diplococcus pneumoniae*, and has obvious antibacterial effects.

11. Effect on ovalbumin (OVA)-induced allergic rhinitis in rats

11.1 Experiment Method

SD rats, all male, were randomly divided into 6 groups according to weight levels: normal control group, model control group, Biyankang 0.4 g/kg group, and a low dose group (37 mg/kg), a medium dose group (74 mg/kg) and a high dose group (148 mg/kg) of the pharmaceutical volatile oil, 15 rats for each group; the rats in the groups, except the normal control group, were intraperitoneally injected with a mixture of 0.3 mg ovalbumin (OAV), 30 mg Al(OH)$_3$ and 1 mL physiological saline, for primary immunization; and on day 15, they were administered in each nostril with 4% OVA 200 $\mu$g (50 $\mu$L) by nasal drip, for boost immunization. The rats in each of the groups were administrated on day 15 of sensitization, once a day, for 14 days. The rats were intragastrically administrated with 10 mL/kg (formulated with soybean oil to the corresponding concentrations), and the rats in the normal control and model control groups were intragastrically administrated with an equal amount of soybean oil.

11.2 Determination of indicators

At 1 hour after the last administration, the rats in each group were intraperitoneally injected with 10% chloral hydrate at 0.35 g/kg for anesthesia; the blood in the rats was sampled from abdominal aorta, and centrifuged at 3000 r/min for 15 min at 4 °C, to separate serum, stored at -70 °C; and histamine (HIS), interleukin-4 (IL-4) and tumor necrosis factor (TNF-$\alpha$) were measured according to the instruction of kits .

11.3 Experimental Results

Compared with the normal control group, the levels of HIS, IL-4 and TNF-$\alpha$ in serum of the rats in the model control group were significantly increased (*P* < 0.01); compared with the model control group, the levels of HIS and IL-4 in serum of the rats in the medium and high dose groups of the pharmaceutical volatile oil were significantly reduced ($P < 0.05$ or $P < 0.01$), and the TNF-$\alpha$ level in serum of the rats in each dose group of the pharmaceutical volatile oil was significantly reduced ($P < 0.05$ or $P < 0.01$). The results were shown in Table 12.

Table 12 Effect of the pharmaceutical volatile oil on OVA-induced allergic rhinitis in rats ($\bar{x} \pm s$, n=15)

| Groups | Dose (mg/kg) | HIS ($\mu$g/L) | IL-4 (ng/L) | TNF-$\alpha$ (ng/L) |
|---|---|---|---|---|
| normal control | - | 71.61±6.33 | 43.22±8.61 | 29.96±8.22 |
| model control | - | 102.55±7.42## | 79.68±5.42## | 63.41±10.52## |
| Biyankang | 400 | 85.14±6.72** | 61.22±5.15** | 39.44±9.08** |
| pharmaceutical volatile oil | 37 | 98.36±6.55 | 72.15±8.21 | 51.28±9.16* |
| | 74 | 95.36±7.11* | 67.98±7.23* | 46.86±7.96** |
| | 148 | 89.02±5.33** | 62.33±5.57** | 45.36±8.17** |

Note: compared with the normal control group, ## $P < 0.01$; compared with the model control group, * $P < 0.05$, "$P < 0.01$.

Under the experiment conditions and the designed dosages in this experiment, the pharmaceutical volatile oil could obviously improve OVA-induced allergic rhinitis in rats, and reduces inflammation reaction.

**Example 4**

The raw material proportion of the pharmaceutical composition was the same as Example 1; the *Herba Pogostemonis* volatile oil, *Rhizoma Atractylodis* volatile oil, *Herba Moslae* volatile oil, *Folium Artemisiae Argyi* volatile oil, *Flos Caryophylli* volatile oil, and *Herba Menthae Haplocalycis* volatile oil were prepared according to the method of Example 1; and they were mixed to give the mixed volatile oil.

200 mL of medium chain triglyceride was taken, heated to 50 °C-60 °C, and added with natural borneol. When the natural borneol completely dissolved, the mixture was cooled down to room temperature, and then added to the mixed volatile oil to give the pharmaceutical volatile oil. The pharmaceutical volatile oil was diluted with medium chain triglycerides to adjust the total amount to 1000 mL, stirred uniformly, and filtrated, to give a light yellow to yellow clear oily solution (having an aromatic odor and slightly bitter taste, and with a relative density (General Notice of Pharmacopoeia 0601) of 0.933 to 0.961); and it is then filled to give the spray, 20 mL for each bottle. When the spray is stored in an airtight place away from light, its effective duration could be 24 months.

Further, the spray was identified:

(1) 15 mL of the product was taken, added with 50 mL petroleum ether (60 °C-90 °C), mixed uniformly, and extracted with 10 mL of 2 mol/L sodium hydroxide solution; the aqueous layer was taken, adjusted to pH of 2.0 with 2 mol/L hydrochloric acid solution, and extracted using 10mL petroleum ether (60 °C-90 °C) with shaking, to separate the petroleum ether layer; and the petroleum ether layer was concentrated to 0.5 mL, as the test solution. The reference substances of patehouli aleohal and pogostone were taken, and added with ethyl acetate respectively to prepare a solution with 4 mg reference substance per 1 mL, as the reference substance solution. According to thin layer chromatography (General Notice of Pharmacopoeia 0502), 2 $\mu$L of the test solution and 1 $\mu$L of the reference substance solution were aspirated, and spotted on the same silica gel G thin layer plate respectively, and the plate was developed with petroleum ether (60 °C-90 °C)-ethyl acetate-formic acid (10:2:1) as the developing solvent; the plate was taken out, dried, soaked in a 5% ferric trichloride solution in ethanol for development, and heated till the spots were clearly visible. In the test sample chromatogram, the same purple blue spots appeared in the position corresponding to the patehouli aleohal reference substance; and in the position corresponding to the pogostone reference substance, the spots with the same color appeared.

(2) Content determination: the determination was performed according to gas chromatography

(General Notice of Pharmacopoeia 0521)

Preparation of a reference substance solution: appropriate amounts of the reference substances of eugenol and patehouli aleohal were precisely weighed, and added with n-hexane respectively, to prepare a solution with 0.3 mg eugenol per 1 mL, and a solution with 0.3 mg patehouli aleohal per 1 mL.

Preparation of the test solution: 1 mL of the product was precisely weighed, placed in a 10 mL volumetric flask, added with n-hexane to the mark, and then shaken uniformly, to give the test solution.

Chromatographic conditions and system suitability tests: a capillary chromatographic column (column length: 30 m, inner diameter: 0.32 mm, thickness: 0.25 $\mu$m), having 100% dimethyl polysiloxane as the stationary phase; column temperature, programmed temperature increase, an initial temperature of 70 °C for 5 min; heating up to 110 °C at 5 °C/min, to 280 °C at 10 °C /min, then keeping for 4min; heating up to 300 °C at 10 °C/min, keeping for 5 min. The theoretical plate number should be not less than 40000 according to the camphol peak. 1 $\mu$L of the reference substance solutions and the test solutions was precisely taken respectively, and injected into a gas chromatograph to measure.

The test chromatogram showed the presence of chromatographic peaks at the retention time corresponding to that of the chromatographic peaks of the patehouli aleohal, carvacrol, eucalyptol, camphol, isoborneol, eugenol, and menthol reference substances.

The product contains not less than 3.75 mg of *Flos Caryophylli,* calculated as eugenol ($C_{10}H_{12}O_2$), per 1 mL; and not less than 5.2 mg of *Herba Pogostemonis,* calculated as patehouli aleohal ($C_{15}H_{26}O$), per 1 mL.

The spray showed the effect of dispelling filth with aroma, diffusing the lung and regulating qi, dispelling dampness and resolving phlegm, and clearing throat. It can be used for the light type, ordinary type, and recovery period of COVID-19, and nasal obstruction, runny nose, cough and sore throat caused by cold or influenza.

It can be directly sprayed to throat or applied to nasal cavity for use. For the light type and ordinary type of COVID-19, it is sprayed to the throat, 2 times each in the morning, afternoon and evening, 6 pressing/spray; for the recovery period of COVID-19, it is sprayed to the throat, once each in the morning, afternoon and evening, 3 pressing/spray; for cough and sore throat caused by cold or influenza, it is sprayed to the throat twice daily, 3 pressing/spray; for nasal obstruction, runny nose, it may be sprayed on the cotton swab and applied to nasal cavity.

*In vitro* anti-SARS-CoV-2 activity tests were performed on the above spray.

1. Toxicity tests of the test sample to VeroE6 cells

1.1 Experimental Method

Vero E6 cells were cultured in DMEM high glucose complete culture medium with no phenol red (cargo no.: E600005-0500, batch no. FC11FC0255, purchased from Sangon Biotech (Shanghai) Co., Ltd.) containing 10% fetal bovine serum (FBS), 100 IU/mL penicillin, and 100 $\mu$g/mL streptomycin. The cells were subcultured once the day before the experiment, so that the cells were in the logarithmic growth phase.

The Vero E6 cells were inoculated in a 96-well plate in $2 \times 10^4$ cell/well, and cultured overnight at 37 °C, 5% $CO_2$; when the monolayer of the cells grew to about 70%, the wells were added with the test samples at different concentrations (in which the spray diluted with DMEM culture medium containing 3% FBS was used, dilution before use) or Redisivir (in which the stock solution was dissolved in DMSO, to the final concentration of 100 mM/mL, stored at 4 °C; the working solution was diluted with DMEM culture medium containing 3% FBS, dilution before use) respectively, 100 $\mu$L/well; three replicate wells were set; meanwhile, the control well without drug was set; the culture was performed for 3 days at 37 °C, 5% $CO_2$.

1.2 Determination of indicators

The survival rate of the cells was detected using CCK8 kit (cargo no.: C0039, Batch no.: 121819200616, purchased from Beyotime Biotechnology Co., Ltd. (Shanghai)). The OD value was determined using Synergy 2 multimode microplate reader, with a measurement wavelength of 450 nm, and a reference wavelength of 630 nm.

According to the experimental results, dose-response fit curves were plotted by Origin7.5, and 50% inhibition concentration for cell growth ($CC_{50}$) of the sample was calculated as the Reed & Muench method. Cell growth survival rate (%) = OD value of experimental well / OD value of control well $\times$ 100.

1.3 Experimental Results

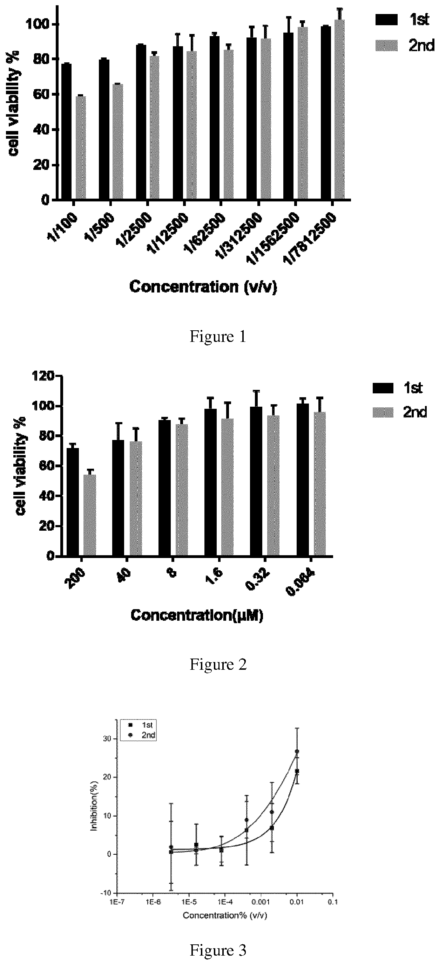

The results were shown in Tables 13 and 14. As shown in FIGs. 1 and 2, the $CC_{50}$ of the spray to Vero E6 cells was > 1/100 (v/v), and the $CC_{50}$ of remdesivir to Vero E6 cells was > 200 $\mu$M.

Table 13 Toxicity of the spray to Vero E6 cells

| Xianghuo spray | Concentration (volume ratio) | Cell survival rate $\pm$ SD (%) | $CC_{50}$ (v/v) |
| --- | --- | --- | --- |
| Test 1 | 1/100 | 77.09$\pm$0.41 | >1/100 |
| | 1/500 | 79.68$\pm$0.41 | |
| | 1/2500 | 87.82$\pm$0.51 | |
| | 1/12500 | 87.25$\pm$7.03 | |
| | 1/62500 | 93.01$\pm$1.94 | |
| | 1/312500 | 92.58$\pm$5.81 | |
| | 1/1562500 | 94.81$\pm$8.97 | |
| | 1/7812500 | 98.34$\pm$0.71 | |
| Test 2 | 1/100 | 58.70$\pm$0.85 | >1/100 |
| | 1/500 | 65.55$\pm$0.30 | |
| | 1/2500 | 81.71$\pm$1.96 | |
| | 1/12500 | 84.48$\pm$9.29 | |
| | 1/62500 | 85.26$\pm$3.06 | |
| | 1/312500 | 91.48$\pm$7.53 | |
| | 1/1562500 | 98.22$\pm$3.11 | |
| | 1/7812500 | 102.41$\pm$6.13 | |

Table 14 Toxicity of remdesivir to Vero E6 celld

| Remdesivir | Concentration ($\mu$M) | Cell survival rate $\pm$ SD (%) | $CC_{50}$ ($\mu$M) |
| --- | --- | --- | --- |
| Test 1 | 200 | 71.58$\pm$3.12 | >200 |
| | 40 | 77.22$\pm$11.23 | |
| | 8 | 90.08$\pm$1.88 | |
| | 1.6 | 98.11$\pm$7.16 | |
| | 0.32 | 99.55$\pm$10.53 | |
| | 0.064 | 101.58$\pm$3.33 | |
| Test 2 | 200 | 53.84$\pm$3.48 | >200 |
| | 40 | 76.24$\pm$8.59 | |
| | 8 | 87.35$\pm$4.25 | |
| | 1.6 | 91.37$\pm$10.73 | |
| | 0.32 | 93.59$\pm$6.96 | |
| | 0.064 | 95.44$\pm$10.01 | |

2 Inhibition tests of the test sample to VeroE6 cell death induced by SARS-CoV-2 (simultaneous addition of drug and virus in the groups of cells)

2.1 Experimental Method

In a P2 laboratory, Vero E6 cells were inoculated in a 96-well plate in $2 \times 10^4$ cell/well, and cultured overnight at 37 °C, 5% $CO_2$; when the monolayer of the cells grew to about 70%, they were transferred to a P3 laboratory for use.

According to the results of the toxicity tests of the test samples and remdesivir to Vero E6 cells, different concentration gradients were set, with three replicate wells for each gradient; in the P3 laboratory, each well in the cell culture plate was added with 50 $\mu$L of the pre-prepared test samples / remdesivir and 50 $\mu$L of virus diluted supernatant (MOI 0.1) simultaneously; a negative control without drug and virus, and a positive control only without drug were set; the cells were cultured for 3 days at 37 °C, 5% $CO_2$.

2.2 Determination of indicators

The cell survival rate was detected with CCK8 kit, and the OD value was determined using a Bio-Tek microplate reader, with a measurement wavelength of 450 nm, and a reference wavelength of 630 nm.

According to the experimental results, dose-response fit curves were plotted by Origin7.5, 50% effective concentration for virus inhibition ($IC_{50}$) of the sample was calculated as the Reed & Muench method, and the therapeutic index TI value for anti SARS-CoV-2 activity was: TI = $CC_{50}$/ $IC_{50}$; inhibition rate (%) to SARS-CoV-2 induced cell death = (OD value of test well - OD value of positive control well) / (OD value of negative control well - OD value of positive control well) $\times$ 100.

2.3 Experimental Results

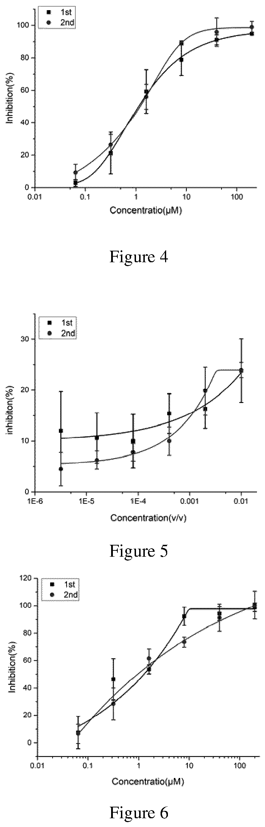

The results were shown in Tables 15 and 16; as shown in FIGs. 3 and 4, the spray group has > 1/100 (v/v) of the $IC_{50}$, and the remdesivir group has 1.118 $\pm$ 0.052 $\mu$M of the $IC_{50}$, TI > 178.89.

Table 15 Inhibition effect of the spray to SARS-CoV-2 induced Vero E6 cell death

| Spray | Concentration (v/v) | Inhibition rate $\pm$ SD (%) | $IC_{50}$ (v/v) |
|---|---|---|---|
| Test 1 | 1/100 | 21.73±3.38 | >1/100 |
| | 1/500 | 6.82±6.38 | |
| | 1/2500 | 6.31±9.02 | |
| | 1/12500 | 0.96±3.82 | |
| | 1/62500 | 2.55±5.31 | |
| | 1/312500 | 0.57±8.06 | |
| Test 2 | 1/100 | 26.67±6.03 | >1/100 |
| | 1/500 | 11.02±7.66 | |
| | 1/2500 | 8.98±4.74 | |
| | 1/12500 | 1.33±3.23 | |
| | 1/62500 | 0.98±0.86 | |
| | 1/312500 | 1.96± 11.27 | |

Table 16 Inhibition effect of remdesivir to SARS-CoV-2 induced Vero E6 cell death

| Remdesivir | Concentration ($\mu$M) | Inhibition rate $\pm$ SD (%) | $IC_{50}$ ($\mu$M) |
|---|---|---|---|
| Test 1 | 200 | 94.82±0.89 | 1.081 |
| | 40 | 91.04±3.06 | |
| | 8 | 78.78±9.66 | |
| | 1.6 | 59.24±13.49 | |
| | 0.32 | 21.29±12.85 | |
| | 0.064 | 2.80±2.11 | |
| Test 2 | 200 | 98.89±3.47 | 1.155 |
| | 40 | 95.81±8.77 | |
| | 8 | 89.19±1.29 | |
| | 1.6 | 55.99±7.66 | |
| | 0.32 | 26.39±6.35 | |
| | 0.064 | 9.23±5.11 | |

3. Inhibition tests of the test sample to VeroE6 cell death induced by SARS-CoV-2 (addition of drug at 1 hour after virus infection of cells)

3.1 Experimental Method

In a P2 laboratory, Vero E6 cells were inoculated in a 96-well plate in 2 $\times$ 10$^4$ cell/well, and cultured overnight at 37 °C, 5% $CO_2$; when the monolayer of the cells grew to about 70%, they were transferred to a P3 laboratory for use.

According to the results of the toxicity tests of the test samples and remdesivir to Vero E6 cells, different concentration gradients were set, with three replicate wells for each gradient; in the P3 laboratory, each well in the cell culture plate was added with 50 $\mu$L of virus diluted supernatant (MOI 0.1); a negative control without drug and virus, and a positive control only without drug were set. After 1 hour incubation in a 5% $CO_2$ incubator at 37 °C, the test wells were added respectively with the pre-prepared test samples / remdesivir, 50 $\mu$L/cell; and the cells were cultured for 3 days at 37 °C, 5% $CO_2$.

3.2 Determination of indicators

The cell survival rate was detected with CCK8 kit, and the OD value was determined using a Bio-Tek microplate reader, with a measurement wavelength of 450 nm, and a reference wavelength of 630 nm.

According to the experimental results, dose-response fit curves were plotted by Origin7.5, 50% effective concentration for virus inhibition ($IC_{50}$) of the sample was calculated as the Reed & Muench method, and the therapeutic index TI value for anti SARS-CoV-2 activity was: TI = $CC_{50}$/ $IC_{50}$; inhibition rate (%) to SARS-CoV-2 induced cell death = (OD value of test well - OD value of positive control well) / (OD value of negative control well - OD value of positive control well) $\times$ 100.

3.3 Experimental Results

The results were shown in Tables 17 and 18; as shown in FIGs. 5 and 6, the spray group has > 1/100 (v/v) of the $IC_{50}$, and the remdesivir group has 0.825 $\pm$ 0.128 $\mu$M of the $IC_{50}$, TI > 242.42.

Table 17 Inhibition effect of the spray to SARS-CoV-2 induced Vero E6 cell death

| Spray | Concentration (v/v) | Inhibition rate $\pm$ SD (%) | $IC_{50}$ (v/v) |
|---|---|---|---|
| Test 1 | 1/100 | 23.83±6.25 | >1/100 |
| | 1/500 | 16.31±3.80 | |
| | 1/2500 | 15.40±3.89 | |
| | 1/12500 | 9.99±5.28 | |
| | 1/62500 | 10.59±4.95 | |
| | 1/312500 | 12.03±7.66 | |
| Test 2 | 1/100 | 23.94±1.50 | >1/100 |
| | 1/500 | 19.80±4.72 | |
| | 1/2500 | 9.99±2.78 | |
| | 1/12500 | 7.80±1.71 | |
| | 1/62500 | 6.26± 1.78 | |
| | 1/312500 | 4.49±3.28 | |

Table 18 Inhibition effect of remdesivir to SARS-CoV-2 induced Vero E6 cell death

| Remdesivir | Concentration ($\mu$M) | Inhibition rate $\pm$ SD (%) | $IC_{50}$ ($\mu$M) |
|---|---|---|---|
| Test 1 | 200 | 98.83±2.97 | 0.915 |
| | 40 | 94.31±5.01 | |
| | 8 | 92.29±6.68 | |
| | 1.6 | 53.50±3.37 | |
| | 0.32 | 46.26±15.18 | |
| | 0.064 | 7.48±11.79 | |
| Test 2 | 200 | 100.47±9.98 | 0.734 |
| | 40 | 91.22±9.91 | |
| | 8 | 73.41±3.67 | |
| | 1.6 | 61.49±6.96 | |
| | 0.32 | 28.39±11.56 | |
| | 0.064 | 6.51±7.08 | |

4 Tests of direct killing of virus

4.1 Experimental Method

In a P2 laboratory, Vero E6 cells were inoculated in a 96-well plate in $2 \times 10^4$ cell/well, and cultured overnight at 37 °C, 5% $CO_2$; when the monolayer of the cells grew to about 70%, they were transferred to a P3 laboratory for use.
In the 96-well plate, 100 $\mu$L of the test sample that has been diluted to 1/100 (v/v), or 0.01% $H_2O_2$ were mixed with 100 $\mu$L of SARS-CoV-2 diluent (MOI 0.1); a positive control without drug and a negative control without virus were set; and the plate was incubated at 37 °C, 5% $CO_2$, for 0, 5 min, 10 min, 30 min and 60 min.
The culture medium in the cell culture plate was aspirated off, the mixed solution of the above drugs and the virus was added respectively, 200 $\mu$L/well, and the plate was cultured for 3 days in a 5% $CO_2$ incubator at 37 °C.

4.2 Determination of indicators

The cell survival rate was detected with CCK8 kit, and the OD value was determined using a Bio-Tek microplate reader, with a measurement wavelength of 450 nm, and a reference wavelength of 630 nm.
Inhibition rate (%) to SARS-CoV-2 induced cell death = (OD value of test well - OD value of positive control well) / (OD value of negative control well - OD value of positive control well) $\times$ 100.

4.3 Experimental Results

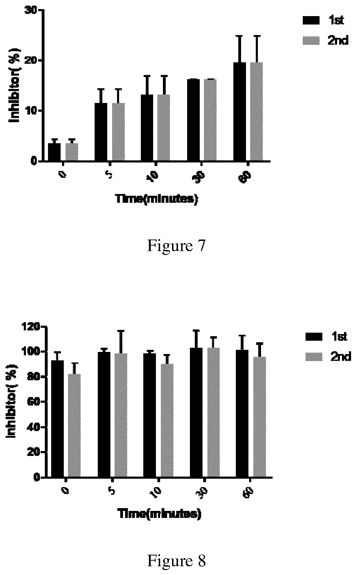

The results were shown in Tables 19 and 20, and FIGs. 7 and 8.

Table 19 Killing effect of the spray to SARS-CoV-2

| Spray (1/100) | Time (min) | Inhibition rate $\pm$ SD (%) |
|---|---|---|
| Test 1 | 0 | 5.24±0.25 |
| | 5 | 11.67±4.21 |
| | 10 | 12.42±7.91 |
| | 30 | 15.36±0.42 |
| | 60 | 13.85±5.47 |
| Test 2 | 0 | 3.50±0.76 |
| | 5 | 11.35±2.83 |
| | 10 | 13.12±3.75 |
| | 30 | 16.14±0.08 |
| | 60 | 19.53±5.35 |

Table 20 Killing effect of $H_2O_2$ (0.01%) to SARS-CoV-2

| $H_2O_2$ (0.01%) | Time (min) | Inhibition rate $\pm$ SD (%) |
|---|---|---|
| Test 1 | 0 | 92.94±6.31 |
| | 5 | 99.25±2.95 |
| | 10 | 98.25±2.36 |
| | 30 | 102.50±14.39 |
| | 60 | 101.35±11.45 |
| Test 2 | 0 | 82.13±8.64 |
| | 5 | 98.16± 18.42 |
| | 10 | 89.87±7.26 |
| | 30 | 102.74±8.56 |
| | 60 | 95.28±10.93 |

In summary, the spray of the present application has an inhibitory effect to SARS-CoV-2 induced Vero E6 cell death when incubated with the cells and virus; when the concentration was 1/100 (v/v), the inhibition rate could reach 26.67%; when the spray was administrated after 1 hour pre-incubation of the cells and virus, it has similar inhibitory effect on SARS-CoV-2 induced Vero E6 cell death to that of the simultaneous incubation group, the inhibition rate at the concentration of 1/100 (v/v) could reach 23.94%; and the spray with the concentration of 1/100 (v/v) has a killing effect on SARS-CoV-2, and the inhibition rate at 60 min was 19.53%.

Example 5

For a pharmaceutical composition, the raw materials comprise 445 g of *Herba Pogostemonis,* 934 g of *Rhizoma Atractylodis,* 532 g of *Herba Moslae,* 1087 g of *Folium Artemisiae Argyi,* 2 g of *Flos Caryophylli*, 745 g of *Herba Menthae Haplocalycis* and 15 g of natural borneol.

The above pharmaceutical composition was prepared into the pharmaceutical volatile oil according to the same method of Example 1.

The pharmaceutical volatile oil was diluted with medium chain triglyceride to adjust the total amount to 1000mL, stirred uniformly, filtrated, filled into a spray bottle according to the specification of 20 mL/bottle, and tightened with a spray pump, to give the spray.

Example 6

For a pharmaceutical composition, the raw materials comprise 885 g of *Herba Pogostemonis,* 1869 g of *Rhizoma Atractylodis,* 1064 g of *Herba Moslae,* 2174 g of *Folium Artemisiae Argyi,* 46 g of *Flos Caryophylli*, and 532 g of *Herba Menthae Haplocalycis.*

*Herba Pogostemonis, Rhizoma Atractylodis, Herba Moslae, Folium Artemisiae Argyi, Flos Caryophylli*, and *Herba Menthae Haplocalycis* were taken, dissected and mixed, added with 8 times their own weight in water and soaked overnight, followed by distilling with steam distillation, and about 14000mL of the distillate was collected, namely the aromatic water. Pharmacodynamic research experiments on the aromatic water were performed:
In the pharmacodynamic research, the doses for mice were 12 mL/kg, 24 mL/kg and 40 mL/kg respectively.

1. Protection effect on virus-induced death of mice

1.1 Experimental Method

Asian mouse-lung-adaptation strain of influenza A virus A/FM/1/34 (H1N1) (FM1) was inoculated in a 9-day-old chick embryo allantoic cavity before using, and conventionally cultured; allantoic fluid was collected freshly and aseptically after 48h, and subcultured twice, to measure the median lethal dose ($LD_{50}$) in ICR mice. ICR mice, half male and half female, were randomly divided into 6 groups according to weight levels: normal control group, model control group, ribavirin 100 mg/kg group, and a low dose group (12 mL/kg), a medium dose group (24 mL/kg) and a high dose group (40 mL/kg) of the aromatic water, 20 mice for each group. The mice were lightly anaesthetized with diethyl ether, and each of the mice was inoculated intranasally with 25 μL of virus fluid, with the infection dose of 25 times $LD_{50}$; while the mice in the normal control group were dropped intranasally with an equal amount of sterile physiological saline. The mice in each dose group of the aromatic water were administrated twice daily, the interval between each administration was 6 hours, and the does for each administration were half of the total dose: 6 mL/kg, 12 mL/kg and 20 mL/kg, and the ribavirin group was administrated by intraperitoneal injection, once a day, for 7 days; and the normal control and model control groups were intragastrically administrated with an equal amount of distilled water.

1.2 Determination of indicators

The disease symptoms of mice were observed daily, and death time, death number and weight were recorded, for 14 days. The mouse that was alive on day 14 was calculated based on 14 days, and the mouse that died within 24 hours post infection was regarded as non-infection death.

1.3 Experimental Results

There is no mouse died in the normal control group, while the survival rate of the mice in the model control group was only 10.0%; and compared with the model control group, the survival rates of the low, medium, and high dose groups of the aromatic water were 30.0%, 40.0%, and 55.0% respectively, which were significantly increased ($P < 0.05$ or $P < 0.01$). The results were shown in Table 21.

Table 21 Effect of the aromatic water on survival rate of influenza virus FM1 infected mice (n=20)

| Groups | Dose (mL/kg) | Survival number | Survival rate (%) |
|---|---|---|---|
| normal control | - | 20 | 100 |
| model control | - | 2 | 10.0## |
| ribavirin | 100 mg/kg | 14 | 70.0** |
| | 12 | 6 | 30.0' |
| aromatic water | 24 | 8 | 40.0* |
| | 40 | 11 | 55.0** |

Note: compared with the normal control group, ## $P < 0.01$; compared with the model control group, * $P < 0.05$, ** $P < 0.01$.

It can be seen from the above table that, the aromatic water could obviously improve the survival rate of influenza virus FM1 infected mice, and has an obvious anti-viral effect.

2. Effect on viral pneumonia of mice infected with influenza virus

2.1 Experimental Method

Asian mouse-lung-adaptation strain of influenza A virus A/FM/1/34 (H1N1) (FM1) was inoculated in a 9-day-old chick embryo allantoic cavity before using, and conventionally cultured; allantoic fluid was collected freshly and aseptically after 48h, and subcultured twice, to measure the median lethal dose ($LD_{50}$) in ICR mice. ICR mice, half male and half female, were randomly divided into 6 groups according to weight levels: normal control group, model control group, oseltamivir 27.5 mg/kg group, and a low dose group (12 mL/kg), a medium dose group (24 mL/kg) and a high dose group (40 mL/kg) of the aromatic water, 14 mice for each group. The mice were lightly anaesthetized with diethyl ether, and each of the mice was inoculated intranasally with 20 $\mu$L of virus fluid, with the infection dose of 25 times $LD_{50}$; while the mice in the normal control group were dropped intranasally with an equal amount of sterile physiological saline. The mice in each dose group of the aromatic water were administrated twice daily for 7 days, the interval between each administration was 6 hours, and the does for each administration were half of the total dose: 6 mL/kg, 12 mL/kg and 20 mL/kg. The normal control and model control groups were intragastrically administrated with an equal amount of distilled water.

2.2 Determination of indicators

At 1 hour after the last administration, the mice were weighed, executed, and dissected to remove the whole lung, and the whole lung was weighed to calculate lung index value and lung index inhibition rate. The lung tissue was prepared with physiological saline into 10% lung tissue homogenate; the prepared lung tissue homogenate was subjected to cryogenic centrifugation, and the content of IL-8 and TNF-$\alpha$ in the lung tissue was determined by ELISA method.

$$\text{Lung index} = [\text{lung weight (g)} / \text{body weight (g)}] \times 100;$$

$$\text{Lung index inhibition rate} = (\text{mean lung index of model control group} - \text{mean lung index of test group}) / \text{mean lung index of model control group}.$$

2.3 Experimental Results

Compared with the normal control group, the lung index of the mice in the model control group was significantly increased ($P < 0.01$); and compared with the model control group, the lung index of each of the dose groups of the aromatic water was significantly reduced ($P < 0.05$ or $P < 0.01$). The results were shown in Table 22.

Table 22 Effect of the aromatic water on lung index of pneumonia mice infected with influenza virus FM1 ($\bar{x}\pm$s, n=14)

| Groups | Dose (mL/kg) | Lung index | Lung index inhibition rate % |
|---|---|---|---|
| normal control | - | 0.67$\pm$0.04 | - |

(continued)

| Groups | Dose (mL/kg) | Lung index | Lung index inhibition rate % |
|---|---|---|---|
| model control | - | $1.35\pm0.18$[##] | - |
| oseltamivir | 27.5 mg/kg | $0.89\pm0.17$** | 34.1 |
| | 12 | $1.30\pm0.12$ | 3.7 |
| aromatic water | 24 | $1.16\pm0.14$* | 14.1 |
| | 40 | $0.94\pm0.19$** | 30.4 |

Note: compared with the normal control group, [##] $P < 0.01$; compared with the model control group, * $P < 0.05$, "$P < 0.01$.

Compared with the normal control group, the content of IL-8 and TNF-$\alpha$ in the mouse lung tissue homogenate of the model control group was significantly increased ($P < 0.01$); compared with the model control group, the content of TNF-$\alpha$ in the mouse lung tissue homogenate of each dose group of the aromatic water was obviously reduced ($P < 0.05$ or $P < 0.01$), and the content of IL-8 in the mouse lung tissue homogenate of the medium and high dose groups of the aromatic water was obviously reduced ($P < 0.05$ or $P < 0.01$). The results were shown in Table 23.

Table 23 Effect of the aromatic water on IL-8 and TNF-$\alpha$ of pneumonia mice infected with influenza virus FM1 ($\bar{x}\pm s$, n=14)

| Groups | Dose (mL/kg) | IL-8 (pg/mL) | TNF-$\alpha$ (pg/mL) |
|---|---|---|---|
| normal control | - | $36.1\pm8.5$ | $51.6\pm18.4$ |
| model control | - | $142.5\pm26.4$[##] | $136.7\pm21.5$[##] |
| oseltamivir | 27.5 mg/kg | $70.6\pm19.2$** | $79.6\pm18.3$** |
| | 12 | $129.8\pm29.6$ | $109.2\pm19.1$* |
| aromatic water | 24 | $115.6\pm18.2$* | $101.5\pm20.5$* |
| | 40 | $94.3\pm17.9$** | $92.6\pm14.3$** |

Note: compared with the normal control group, [##] $P < 0.01$; compared with the model control group, * $P < 0.05$, "$P < 0.01$.

In summary, the aromatic water could obviously improve viral pneumonia induced by H1N1 influenza.

3. Protection effect on mice infected with *Diplococcus pneumoniae*

3.1 Experimental Method

ICR mice, half male and half female, were randomly divided into 6 groups according to weight levels: normal control group, model control group, Shuanghuanglian 20 g/kg group, and a low dose group (12 mL/kg), a medium dose group (24 mL/kg) and a high dose group (40 mL/kg) of the aromatic water, 20 mice for each group. The mice in each dose group of the aromatic water were administrated twice daily for 7 days, the interval between each administration was 6 hours, and the does for each administration were half of the total dose: 6 mL/kg, 12 mL/kg and 20 mL/kg. The normal control and model control groups were intragastrically administrated with an equal amount of distilled water.

3.2 Determination of indicators

At 1 hour after administration on day 4, the mice, except those in the normal control group, were intraperitoneally injected with a solution of *Diplococcus pneumoniae* at 0.5mL/mouse for 3 days, and the death of mice in each group within 7 days was observed.

3.3 Experimental Results

Compared with the normal control group, the death of the mice infected with *Diplococcus pneumoniae* was obviously increased in 7 days, with the survival rate of 20% ($P < 0.01$); and compared with the model control group, the mortality of the mice in the medium and high dose groups of the pharmaceutical volatile oil in 7 days was obviously reduced ($P < 0.05$ or $P < 0.01$). The results were shown in Table 24.

Table 24 Effect of the aromatic water on survival rate of mice infected with *Diplococcus pneumoniae* (n=20)

| Groups | Dose (mL/kg) | Survival number | Survival rate (%) |
|---|---|---|---|
| normal control | - | 20 | 100 |
| model control | - | 3 | 15.0## |
| Shuanghuanglian | 20 g/kg | 12 | 60.0** |
| | 12 | 6 | 30.0 |
| aromatic water | 24 | 9 | 45.0* |
| | 40 | 11 | 55.0** |

Note: compared with the normal control group, ## *P* < 0.01; compared with the model control group, * *P* < 0.05, "*P* < 0.01.

It can be seen from the above table, the aromatic water could obviously improve the survival rate of mice infected with *Diplococcus pneumoniae*, and has an obvious antibacterial effect.

**Example 7**

For a pharmaceutical composition, the raw materials comprise 885 g of *Herba Pogostemonis,* 1869 g of *Rhizoma Atractylodis,* 1064 g of *Herba Moslae,* 2174 g of *Folium Artemisiae Argyi,* 46 g of *Flos Caryophylli*, 532 g of *Herba Menthae Haplocalycis*, 35 g of sucralose and 35 g of sodium benzoate.

*Herba Pogostemonis, Rhizoma Atractylodis, Herba Moslae, Folium Artemisiae Argyi, Flos Caryophylli*, and *Herba Menthae Haplocalycis* were weighed, dissected and mixed, added with 8 times their own weight in water and soaked overnight, followed by distilling with steam distillation; the distillate was collected, added with sucralose and sodium benzoate, heated (not above 60 °C), stirred uniformly, and filled according to the specification of 20mL per bottle, and sealed; after filling, sterilization was performed for 30 min at 121 °C by moist heat sterilization, to give the distillate medicinal water.

**Example 8**

The pharmaceutical volatile oil was prepared according to the method of Example 1; the pharmaceutical volatile oil was diluted with anhydrous ethanol to adjust the total amount to 1000mL, stirred uniformly, filtrated, filled for example into a pressure-resistant aluminium bottle according to the specification of 20 mL/bottle, capped, and filled with a certain amount of nitrogen gas to the pressure of 0.8 Mpa in the aluminium bottle, to give the aerosol.

**Example 9**

For an antibacterial and antiviral pharmaceutical composition, the raw materials comprise 960 g of *Herba Pogostemonis,* 1920 g of *Rhizoma Atractylodis,* 1200 g of *Herba Moslae,* 2160 g of *Folium Artemisiae Argyi,* 240 g of *Flos Caryophylli,* 720 g of *Herba Menthae Haplocalycis* and 10 g of natural borneol.

The above pharmaceutical composition was used for preparing the pharmaceutical volatile oil, and the preparation method was the same as Example 1.

The preparation method for the pharmaceutical volatile oil dropping pill was as follows:

A. the emulsifier, corn protein, and sunflower phospholipid were mixed in the prepared pharmaceutical volatile oil, wherein the mass fractions thereof in the pharmaceutical volatile oil are 1%, 1.5% and 0.5%, respectively;

B. starch sodium octenyl succinate was dissolved in water, to prepare a aqueous solution with a mass fraction of 20%;

C. the two solutions prepared in A and B were mixed according in the weight ratio of 1:2, stirred and emulsified by using a 12000r/min ultra-high speed stirrer, and homogenized 3 times using a high pressure homogenizer;

D. the homogenized solution obtained in C was spray-dried to give the volatile oil microcapsule powder; and

E. the volatile oil microcapsule powder and a dropping pill matrix in the mass ratio of 1:2 were prepared into dropping pills, wherein the dropping pill matrix material is polyethylene glycol 4000 : polyethylene glycol 6000 (1:1).

Each pill is 0.32 g, and contains 7 mg of the pharmaceutical volatile oil. When application in the treatment of the mild type, ordinary type, or a recovery period of COVID-19, or nasal obstruction, runny nose, cough and sore throat caused by cold or influenza, and the like, the pills were taken three times a day in the morning/afternoon/evening, 14 pills for one time.

The pharmacodynamic experiments were performed on the pharmaceutical volatile oil dropping pill of this example. For the experimental groups, the doses were 1.10 g/kg for mice, 0.56 g/kg for rats, and 0.48 g/kg for guinea pigs in the experiments. Control groups 1, 2 and 3 were set. Among them, the dropping pills in Control group 1 were prepared according to the method of the experimental groups, except that *Folium Artemisiae Argyi* was replaced with *Herba Ephedra*, *Herba Menthae Haplocalycis* was replaced with *Radix Angelicae Dahuricae,* and *Flos Caryophylli* and natural borneol were not added.

The dropping pills in Control group 2 were prepared according to the method of the experimental groups, except that *Herba Pogostemonis* was replaced with *Radix Isatidis*, and *Rhizoma Atractylodis* was replaced with *Radix Saposhnikoviae.*

The dropping pills in Control group 3 were prepared according to the method of the experimental groups, except that *Herba Moslae* was replaced with *Fructus Gardeniae*, and *Folium Artemisiae Argyi* was replaced with *Pericarpium Citri Reticulatae.*

1. Concentrated ammonia water induced cough tests in mice (relieving cough effects)

1.1 Experimental Method

ICR mice were used, half male and half female. The mice were randomly divided into 7 groups according to weight levels: normal control group, model control group, pentoxyverine citrate 20 mg/kg group, the pharmaceutical volatile oil dropping pill group, control group 1, control group 2, and control group 3, 14 mice for each group. The mice were intragastrically administrated with 20 mL/kg (the dropping pills were ground into powder, and suspended with 0.5% CMC-Na to the corresponding concentrations in each administration group), once a day, for 7 days. The mice in the normal control and model control groups were intragastrically administrated with an equal amount of a 0.5% CMC-Na solution.

1.2 Determination of indicators

At 1 hour after the last administration, the mice were put in a 500mL transparent sealed container, and the atomized concentrated ammonia water was uniformly sprayed into the container using an ultrasonic atomizer, continuously for 20s. The phenomenon of shrinking abdomen and opening mouth in mice was taken as cough, and cough incubation period and cough frequency of mice within 2 min after being put in the container were recorded.

1.3 Experimental Results

Compared with the model control group, the cough incubation period of the mice in the pharmaceutical volatile oil dropping pill group, and control groups 1, 2 and 3 was significantly prolonged; and the cough frequency of the mice was significantly reduced (P< 0.01); compared with the pharmaceutical volatile oil dropping pill group, the cough incubation period of the mice in control groups 1, 2 and 3 was significantly reduced, and the cough frequency of the mice was significantly increased (P< 0.05). The results were shown in Table 25.

Table 25 Effect of the pharmaceutical volatile oil dropping pill and control groups on concentrated ammonia water induced cough in mice ($\bar{x}\pm$s, n=14)

| Groups | Dose (g/kg) | Cough incubation period (s) | Frequency of cough (time/2 min) |
| --- | --- | --- | --- |
| normal control | - | 0±0 | 0±0 |
| model control | - | 11.2±3.1 | 90.3±15.5 |
| pentoxyverine citrate | 0.02 | 23.3±4.2** | 40.6±12.5** |
| pharmaceutical volatile oil dropping pill | 1.10 | 22.5±4.1** | 46.2±16.9** |
| control group 1 | 1.10 | 16.4±4.2*# | 60.5±18.9*# |
| control group 2 | 1.10 | 17.0±5.1*# | 65.4±20.1*# |

(continued)

| Groups | Dose (g/kg) | Cough incubation period (s) | Frequency of cough (time/2 min) |
|---|---|---|---|
| control group 3 | 1.10 | 16.8±4.6*# | 68.6±23.5*# |

Note: compared with the model control group, * P < 0.05, ** P < 0.01; compared with the pharmaceutical volatile oil dropping pill group, # P < 0.05.

Under the experimental conditions and the designed dosages in this experiment, the pharmaceutical volatile oil dropping pill group, and control groups 1, 2 and 3 have an obvious effect of relieving cough, while the relieving cough effect of the pharmaceutical volatile oil dropping pill group is significantly superior to those of control groups 1, 2 and 3.

2. Effect on asthmatic guinea pigs induced by acetylcholine combined with histamine phosphate (antiasthmatic effect)

2.1 Experimental Method

Conventional guinea pigs were used, half male and half female. The guinea pigs were put into a glass cover (4L volume) one by one, and a mixed solution of equal volumes of 0.1% histamine phosphate and 2% acetylcholine chloride was sprayed into the glass cover using an ultrasonic atomizer for 15s; and pre-administration asthma incubation period of guinea pigs (time from spray to convulsion) was recorded one by one. The guinea pigs with the asthma incubation period of not more than 120s were considered as qualified guinea pigs, while those beyond the asthma incubation period were considered as not sensitive, and were removed. The selected qualified guinea pigs were randomly divided into 6 groups according to the pre-administration asthma incubation period: model control group, aminophylline 60 mg/kg group, the pharmaceutical volatile oil dropping pill group, control group 1, control group 2, and control group 3, 12 guinea pigs for each group. The guinea pigs were intragastrically administrated with 4 mL/kg (the dropping pills were ground into powder, and suspended with 0.5% CMC-Na to the corresponding concentrations in each administration group), once a day, for 7 days. The guinea pigs in the model control group were intragastrically administrated with an equal amount of a 0.5% CMC-Na solution.

2.2 Determination of indicators

At 1 hour after the last administration, the guinea pigs were recorded for the post-administration asthma incubation period using the same method. If the guinea pigs did not show asthma symptoms within 3 min, they were then calculated as 180s.

2.3 Experimental Results

Compared with the model control group, the post-administration asthma incubation period and the extension value of the post-administration asthma incubation period (post-administration incubation period - pre-administration incubation period) were significantly increased in the guinea pigs in the pharmaceutical volatile oil dropping pill group, and control groups 1, 2 and 3 (P < 0.01); and compared with the pharmaceutical volatile oil dropping pill group, the post-administration asthma incubation period and the extension value of the post-administration asthma incubation period (post-administration incubation period - pre-administration incubation period) were significantly reduced in the guinea pigs in control groups 1, 2 and 3 (P< 0.05). The results were shown in Table 26.

Table 26 Effect of the pharmaceutical volatile oil dropping pill and control groups on asthma incubation period of guinea pigs induced by acetylcholine and histamine phosphate ($\bar{x}$±s, n=12)

| Groups | Dose (g/kg) | Cough incubation period (s) | | |
|---|---|---|---|---|
| | | pre-administration | post-administration | extension value (post - pre) |
| model control | - | 37.3±6.2 | 38.4±5.9 | 1.1±7.9 |
| aminophylline | 0.06 | 36.9±5.8 | 95.3±37.6** | 58.4±32.1** |
| pharmaceutical volatile oil dropping pill | 0.48 | 37.1±7.1 | 80.5±29.5** | 43.4±25.8** |
| control group 1 | 0.48 | 37.6±6.9 | 60.8±11.2**# | 23.2±22.1**# |

(continued)

| Groups | Dose (g/kg) | Cough incubation period (s) | | |
|---|---|---|---|---|
| | | pre-administration | post-administration | extension value (post - pre) |
| control group 2 | 0.48 | 37.7±5.7 | 65.1±15.1**# | 27.4±25.4**# |
| control group 3 | 0.48 | 37.4±5.9 | 63.5±17.3**# | 26.1±24.9**# |

Note: compared with the model control group, ** *P* < 0.01; compared with the pharmaceutical volatile oil dropping pill group, # *P* < 0.05.

Under the experiment conditions and the designed dosages in this experiment, the pharmaceutical volatile oil dropping pill group and control groups 1, 2 and 3 have an obvious antiasthmatic effect, while the antiasthmatic effect of the pharmaceutical volatile oil dropping pill group is obviously superior to those of control groups 1, 2 and 3.

3. Effect on capillary discharge sputum volume in rats (phlegm-reducing effect)

3.1 Experimental Method

SD rats, half male and half female, were randomly divided into 6 groups according to weight levels: model control group, carbocisteine 0.2 g/kg group, the pharmaceutical volatile oil dropping pill group, control group 1, control group 2, and control group 3, 12 rats for each group. The rats were intragastrically administrated with 10 mL/kg (the dropping pills were ground into powder, and suspended with 0.5% CMC-Na to the corresponding concentrations in each administration group), once a day, for 7 days. The rats in the model control group were intragastrically administrated with an equal amount of a 0.5% CMC-Na solution.

3.2 Determination of indicators

The rats were anesthetized and fixed in supine position at 40 min after the last administration, and the rats were cut the skin of the middle of neck, to separate the trachea; a glass capillary was inserted through a small hole pricked by a sharp injection needle between the two cricoid cartilages in the middle of the lower edge of the thyroid cartilage, to allow the capillary to just contact the bottom surface of the trachea; the sputum in the trachea was absorbed into the capillary; when the capillary was filled with sputum, it is immediately replaced with another capillary having the same diameter; after continuous collection for 2h, the liquid column in the capillary was measured in length with a graduated scale, to evaluate the sputum excretion volume.

3.3 Experimental Results

Compared with the model control group, the sputum excretion volume of the rats in the pharmaceutical volatile oil dropping pill group, and control groups 1, 2 and 3 was significantly increased (*P*< 0.05 or *P*< 0.01); and compared with the pharmaceutical volatile oil dropping pill group, the sputum excretion volume of the rats in control groups 1, 2 and 3 was significantly reduced (*P*< 0.05). The results were shown in Table 27.

Table 27 Effect of the pharmaceutical volatile oil dropping pill and control groups on capillary discharge sputum volume in rats ($\bar{x}$±s, n=12)

| Groups | Dose (g/kg) | Sputum excretion volume (cm) |
|---|---|---|
| model control | - | 10.52±2.43 |
| carbocisteine | 0.2 | 21.56±4.41** |
| pharmaceutical volatile oil dropping pill | 0.56 | 19.12±3.98** |
| control group 1 | 0.56 | 14.85±3.15*# |
| control group 2 | 0.56 | 15.02±3.65*# |
| control group 3 | 0.56 | 14.66±3.18*# |

Note: compared with the model control group, * *P* < 0.05, ** *P* < 0.01; compared with the pharmaceutical volatile oil dropping pill group, # *P* < 0.05.

Under the experimental conditions and the designed dosages in this experiment, the pharmaceutical volatile oil dropping pill group, and control groups 1, 2 and 3 have an obvious phlegm-reducing effect, while the phlegm-reducing effect of the pharmaceutical volatile oil dropping pill group is obviously superior to those of control groups 1, 2 and 3.

4. Protection effect on mice infected with *Diplococcus pneumoniae* (antibacterial effect)

4.1 Experimental Method

ICR mice, half male and half female, were randomly divided into 7 groups according to weight levels: normal control group, model control group, Shuanghuanglian 20 g/kg group, the pharmaceutical volatile oil dropping pill group, control group 1, control group 2, and control group 3, 20 mice for each group. The mice were intragastrically administrated with 20 mL/kg (the dropping pills were ground into powder and suspended with 0.5% CMC-Na to the corresponding concentrations in each administration group), once a day, for 7 days. The mice in the model control group were intragastrically administrated with an equal amount of a 0.5% CMC-Na solution.

4.2 Determination of indicators

At 1 hour after administration on day 4, the mice, except those in the normal control group, were intraperitoneally injected with a solution of *Diplococcus pneumoniae* at 0.5 mL/mouse for 3 days, and the death of mice in each group within 7 days was observed.

4.3 Experimental Results

Compared with the normal control group, the death of the mice infected with *Diplococcus pneumoniae* was significantly increased in 7 days, with the survival rate of 20% (*P*< 0.01); and compared with the model control group, the mortality of the mice in the pharmaceutical volatile oil dropping pill group, and control groups 1, 2 and 3 in 7 days was significantly reduced (*P*< 0.05). Compared with the pharmaceutical volatile oil dropping pill group, the mortality of the mice in control groups 1, 2 and 3 in 7 days was increased but no statistical difference (*P*> 0.05). The results were shown in Table 28.

Table 28 Effect of the pharmaceutical volatile oil dropping pill and control groups on survival rate of mice infected with *Diplococcus pneumoniae* (n=20)

| Groups | Dose (g/kg) | Survival number | Survival rate (%) |
|---|---|---|---|
| normal control | - | 20 | 100 |
| model control | - | 4 | 20.0## |
| Shuanghuanglian | 20 | 14 | 70.0** |
| pharmaceutical volatile oil dropping pill | 1.10 | 11 | 55.0* |
| control group 1 | 1.10 | 9 | 45.0* |
| control group 2 | 1.10 | 9 | 45.0* |
| control group 3 | 1.10 | 10 | 50.0' |

Note: compared with the normal control group, ## *P* < 0.01; compared with the model control group, * *P* < 0.05.

Under the experimental conditions and the designed dosages in this experiment, the pharmaceutical volatile oil dropping pill group, and control groups 1, 2 and 3 could obviously improve the survival rate of mice infected with *Diplococcus pneumoniae,* and have obvious antibacterial effects. The antibacterial effect of the pharmaceutical volatile oil dropping pill group was slightly better than those of control groups 1, 2 and 3.

5. Comparative study of protection effect on virus-induced death in mice 5.1 Experimental Method

Asian mouse-lung-adaptation strain of influenza A virus A/FM/1/34 (H1N1) (FM1) was inoculated in a 9-day-old chick embryo allantoic cavity before using, and conventionally cultured; allantoic fluid was collected freshly and aseptically after 48h, and subcultured twice, to measure the median lethal dose ($LD_{50}$) in ICR mice. ICR mice, half male and half female, were randomly divided into 6 groups according to weight levels: normal control group, model control group, ribavirin 100 mg/kg group, the pharmaceutical volatile oil dropping pill 1.10 g/kg group, commercial dropping pill 1.73 g/kg group, and commercial spray 20 mL/kg group (maximum administration dosage), 24 mice for each group. The mice were lightly anaesthetized with diethyl ether, and each of the mice was inoculated intranasally with 25 μL of virus fluid, with the infection dose of 25 times $LD_{50}$; while the mice in the normal control group were dropped intranasally with an equal amount of sterile physiological saline. The mice in the pharmaceutical volatile oil dropping pill group, commercial dropping pill group, and commercial spray group were intragastrically administrated with 20 mL/kg (the dropping pills were ground into powder, and suspended with 0.5% CMC-Na to the corresponding concentrations in each administration group), and the mice in the ribavirin group was administrated by intraperitoneal injection, once a day, for 7 days. The mice in the normal control and model control groups were intragastrically administrated with an equal amount of a 0.5% CMC-Na solution.

Wherein, the commercial dropping pills comprises the following pharmaceutical ingredients: cholic acid, mother-of-pearl, Hyodeoxycholic Acid, *Gardenia jasminoides*, *Cornu bubali, Radix Isatidis,* baicalin, and honeysuckle flower; and the commercial spray comprises the following pharmaceutical ingredients: blumea balsamifera, oleum cinnamomi oil, and menthol.

5.2 Determination of indicators

The disease symptoms of the mice were observed daily, and death time, death number and weight were recorded, for 14 days. The mouse that was alive on day 14 was calculated based on 14 days, and the mouse that died within 24 hours post infection was regarded as non-infection death.

10.3 Experimental Results

The mice in the normal control group were in good spirits and agility, and have smooth and shiny fur, normal breathing frequency, normal food and water intake and increased weight daily. The mice infected with influenza virus showed head-twitches, rapid breathing rate, mainly abdominal breathing, slowing in action, curling, shrugged hair, and significant decrease of the food and water intake amount, at 24 hours post infection. Starting from day 4, the mice began to death (reaching a peak on day 7), and the states of the mice in each treatment group were obviously better than those in the model group.

There is no mouse died in the normal control group, while the survival rate of the mice in the model control group was only 6.2%; compared with the model control group, the survival rates of the mice in the pharmaceutical volatile oil dropping pill group, commercial dropping pill group and commercial spray group were 62.5%, 41.7% and 37.5% respectively, which were significantly increased ($P < 0.05$ or $P < 0.01$); and compared with the pharmaceutical volatile oil dropping pill group, the survival rates of the mice of the commercial dropping pill and commercial spray groups were reduced significantly ($P < 0.05$ or $P < 0.01$). The results were shown in Table 29.

Table 29 Effect of the pharmaceutical volatile oil dropping pill, Qingkailing and Jinhoujian on survival rate of influenza virus FM1 infected mice (n=24)

| Groups | Dose | Survival number | Survival rate (%) |
|---|---|---|---|
| normal control | - | 24 | 100 |
| model control | - | 2 | 8.3## |
| ribavirin | 100 mg/kg | 19 | 79.2" |
| pharmaceutical volatile oil dropping pill | 1.10 g/kg | 15 | 62.5" |
| commercial dropping pill | 1.73 g/kg | 10 | 41.7**Δ |
| commercial spray | 20 mL/kg | 9 | 37.5*ΔΔ |

Note: compared with the normal control group, ## $P < 0.01$; compared with the model control group, * $P < 0.05$, ** $P < 0.01$; compared with the pharmaceutical volatile oil dropping pill group, ΔP< 0.05, ΔΔP< 0.01.

Under the experimental conditions and the designed dosages in this experiment, the pharmaceutical volatile oil dropping pill group, commercial dropping pill and commercial spray could significantly improve the survival rate of influenza virus FM1 infected mice, and the anti-viral effect of the pharmaceutical volatile oil dropping pill was significantly superior to those of the commercial dropping pill and commercial spray.

6. Comparative study on viral pneumonia of mice infected with influenza virus

6.1 Experimental Method

Asian mouse-lung-adaptation strain of influenza A virus A/FM/1/34 (H1N1) (FM1) was inoculated in a 9-day-old chick embryo allantoic cavity before using, and conventionally cultured; allantoic fluid was collected freshly and aseptically after 48h, and subcultured twice, to measure the median lethal dose ($LD_{50}$) in ICR mice. ICR mice, half male and half female, were randomly divided into 6 groups according to weight levels: normal control group, model control group, oseltamivir 27.5 mg/kg group, the pharmaceutical volatile oil dropping pill 1.10 g/kg group, commercial dropping pill 1.73 g/kg group, and commercial spray 20 mL/kg group (maximum administration dosage), 14 mice of each group. The mice were lightly anaesthetized with diethyl ether, and each of the mice was inoculated intranasally with 20 $\mu$L of virus fluid, with the infection dose of 25 times $LD_{50}$; while the mice in the normal control group were dropped intranasally with an equal amount of sterile physiological saline. The mice in the pharmaceutical volatile oil dropping pill group, commercial dropping pill group, and commercial spray group were intragastrically administrated with 20 mL/kg, and the mice in the ribavirin group was administrated by intraperitoneal injection, once a day, for 7 days. The mice in the normal control and model control groups were intragastrically administrated with an equal amount of a 0.5% CMC-Na solution.

6.2 Determination of indicators

At 1 hour after the last administration, the mice were weighed, executed and dissected to remove the whole lung, and the whole lung was weighed to calculate lung index value and lung index inhibition rate. The lung tissue was prepared with physiological saline into 10% lung tissue homogenate; the prepared homogenate was subjected to cryogenic centrifugation, and the content of IL-8 and TNF-$\alpha$ in the lung tissue was determined by ELISA method.

$$\text{Lung index} = [\text{lung weight (g)} / \text{body weight (g)}] \times 100;$$

$$\text{Lung index inhibition rate} = (\text{mean lung index of model control group} - \text{mean lung index of test group}) / \text{mean lung index of model control group.}$$

6.3 Experimental Results

Compared with the normal control group, the lung index of the mice in the model control group was significantly increased ($P < 0.01$); compared with the model control group, the lung indexes of the mice in the pharmaceutical volatile oil dropping pill, commercial dropping pill and commercial spray groups were significantly reduced ($P < 0.05$ or $P < 0.01$); and compared with the pharmaceutical volatile oil dropping pill group, the lung indexes of the mice in the commercial dropping pill and commercial spray groups were significantly increased ($P < 0.05$). The results were shown in Table 30.

Table 30 Effect of the pharmaceutical volatile oil dropping pill, Qingkailing and Jinhoujian on lung index of mice ($\overline{x} \pm s$, n=14)

| Groups | Dose | Lung index | Lung index inhibition rate % |
|---|---|---|---|
| normal control | - | 0.69±0.04 | - |
| model control | - | 1.71±0.21## | - |
| oseltamivir | 27.5 mg/kg | 0.89±0.20** | 47.9 |
| pharmaceutical volatile oil dropping pill | 1.10 g/kg | 1.11±0.12** | 35.1 |
| commercial dropping pill | 1.73 g/kg | 1.30±0.15*Δ | 24.0 |
| commercial spray | 20 mL/kg | 1.45±0.17*Δ | 15.2 |

Note: compared with the normal control group, ## $P < 0.01$; compared with the model control group, * $P < 0.05$, ** $P < 0.01$; compared with the pharmaceutical volatile oil dropping pill group, Δ$P < 0.05$.

Compared with the normal control group, the content of IL-8 and TNF-$\alpha$ in the mouse lung tissue homogenate of the model control group was significantly increased ($P < 0.01$); compared with the model control group, the content of IL-8 and TNF-$\alpha$ in the mouse lung tissue homogenate of the pharmaceutical volatile oil dropping pill, commercial dropping pill and commercial spray groups was significantly reduced ($P < 0.05$ or $P < 0.01$); and compared with the pharmaceutical volatile oil dropping pill group, the content of IL-8 and TNF-$\alpha$ in the mouse lung tissue homogenate of the commercial spray group was significantly increased ($P < 0.05$). The results were shown in Table 31.

Table 31 Effect of the pharmaceutical volatile oil dropping pill, Qingkailing and Jinhoujian on IL-8 and TNF-$\alpha$ of pneumonia mice ($\bar{x}\pm$s, n=14)

| Groups | Dose | IL-8 (pg/mL) | TNF-$\alpha$ (pg/mL) |
|---|---|---|---|
| normal control | - | 45.5$\pm$8.3 | 31.2$\pm$11.3 |
| model control | - | 190.3$\pm$33.2## | 135.2$\pm$22.1## |
| oseltamivir | 27.5 | 80.3$\pm$15.3** | 65.2$\pm$17.5** |
| pharmaceutical volatile oil dropping pill | 1.10 g/kg | 102.5$\pm$23.4** | 78.5$\pm$19.9** |
| commercial dropping pill | 1.73 g/kg | 124.8$\pm$20.7* | 92.4$\pm$28.5* |
| commercial spray | 20 mL/kg | 145.5$\pm$21.1*$\Delta$ | 102.7$\pm$23.1*$\Delta$ |

Note: compared with the normal control group, ## $P < 0.01$; compared with the model control group, * $P< 0.05$, ** $P< 0.01$; compared with the pharmaceutical volatile oil dropping pill group, $\Delta P< 0.05$.

Under the experimental conditions and the designed dosages in this experiment, the pharmaceutical volatile oil dropping pill, commercial dropping pill and commercial spray could obviously improve viral pneumonia caused by H1N1 influenza, and the anti-viral effect of the pharmaceutical volatile oil dropping pill was significantly superior to those of the commercial dropping pill and commercial spray.

**Example 10**

The preparation method of the soft capsule of the pharmaceutical volatile oil was as follows:

(1) Preparation of the pharmaceutical volatile oil

The *Herba Pogostemonis* volatile oil, *Rhizoma Atractylodis* volatile oil, *Herba Moslae* volatile oil, *Folium Artemisiae Argyi* volatile oil, *Flos Caryophylli* volatile oil, and *Herba Menthae Haplocalycis volatile* oil were prepared respectively according to the method of Example 1. 20 g of *Herba Pogostemonis* volatile oil, 20 g of *Rhizoma Atractylodis* volatile oil, 10 g of *Herba Moslae* volatile oil, 10 g of *Folium Artemisiae Argyivolatile* oil, 5 g of *Flos Caryophylli* volatile oil, and 5 g of *Herba Menthae Haplocalycis* volatile oil were weighed and mixed uniformly, to give the mixed volatile oil for use.
10 g of Natural borneol was weighed and placed in a vessel having a suitable volume, added with 200 mL medium chain triglycerides, stirred and heated (not above 60 °C) to accelerate the dissolution of the natural borneol, till the natural borneol completely dissolved, for further use. The mixed volatile oil was added to the solution of the natural borneol in middle chain triglycerides, stirred, then added with medium chain triglycerides for up to 1000mL, and mixed uniformly, for further use.

(2) Preparation of capsule materials

Gelatin, glycerol, and purified water were weighed in the mass ratio of 1:1:0.5, placed in a barrel for glue melting, melted and mixed at 70 °C, which was vacuum degassed for further use.

(3) Preparation of the soft capsules

Using YWJ100-IP soft capsule production line, the soft capsules were prepared, each soft capsule containing 0.3 g of the content by adjusting the parameters in the soft capsule machine, and the soft capsules of the pharmaceutical volatile oil were thus obtained.

**Example 11**

The preparation method of the nasal drop of the pharmaceutical volatile oil was as follows:

The raw material proportion of the pharmaceutical composition was the same as Example 1, and the *Herba Pogostemonis* volatile oil, *Rhizoma Atractylodis* volatile oil, *Herba Moslae* volatile oil, *Folium Artemisiae Argyivolatile* oil, *Flos Caryophylli* volatile oil, and *Herba Menthae Haplocalycis* volatile oil were prepared according to the method of Example 1, and then mixed to give the mixed volatile oil. 7 g of the mixed volatile oil was added with 1 g of natural borneol, stirred and properly heated (not above 60 °C) to completely dissolve the natural borneol, to give the pharmaceutical volatile oil, as solution A.

100 mL of a 10% ethyl cellulose solution in anhydrous ethanol was prepared as solution B. Solutions A and B were mixed, added with 100 mL of medium chain triglycerides, stirred and mixed for 30 min, and dried under reduced pressure for removal of anhydrous ethanol, to give a oily gel in light yellow to yellow.

**Example 12**

The preparation method of the preparation for atomization inhalation of the pharmaceutical volatile oil was as follows:

The raw material proportion of the pharmaceutical composition was the same as Example 1, and the *Herba Pogostemonis* volatile oil, *Rhizoma Atractylodis* volatile oil, *Herba Moslae* volatile oil, *Folium Artemisiae Argyivolatile* oil, *Flos Caryophylli* volatile oil, and *Herba Menthae Haplocalycis* volatile oil were prepared according to the method of Example 1, and then mixed to give the mixed volatile oil. 70 g of the mixed volatile oil was weighed, added with 10 g of natural borneol, properly heated (not above 60 °C) and stirred till the natural borneol completely dissolved, to give the pharmaceutical volatile oil.

The pharmaceutical volatile oil was diluted with medium chain triglycerides to adjust the total amount to 1000 mL, stirred uniformly, and filtrated, to give a light yellow to yellow clear oily solution (having an aromatic odor and slightly bitter taste, and with a relative density (General Notice of Pharmacopoeia 0601) of 0.933 to 0.961); and it is then filled, 20 mL for each bottle. The medicine liquid was poured into an atomizer for atomization and inhalation, when it is used. The examples described above are only a part of embodiments of the present application, not all embodiments. Based on the examples of the present application, all other examples obtained by those skilled in the art without creative labor belong to the protection cope of the present application.

**Claims**

1. An antibacterial and antiviral pharmaceutical composition, comprising the following raw materials in parts by weight: 4-14 parts of *Herba Pogostemonis,* 9-28 parts of *Rhizoma Atractylodis,* 5-18 parts of *Herba Moslae,* 11-33 parts of *Folium Artemisiae Argyi,* 0.1-3 parts of *Flos Caryophylli,* and 2-10 parts of *Herba Menthae Haplocalycis.*

2. The antibacterial and antiviral pharmaceutical composition according to claim 1, wherein it further comprises, in parts by weight, 0.01-0.2 parts of natural borneol.

3. The antibacterial and antiviral pharmaceutical composition according to claim 1 or 2, wherein it further comprises a pharmaceutical excipient.

4. An antibacterial and antiviral pharmaceutical volatile oil, wherein the following steps are comprised:

   1) adding water to *Herba Pogostemonis, Rhizoma Atractylodis, Herba Moslae, Folium Artemisiae Argyi, Flos Caryophylli*, and *Herba Menthae Haplocalycis* according to claim 2 respectively, and distilling for extraction of volatile oil, to give the volatile oil of each of the raw materials, and then mixing the volatile oil of each of the raw materials to give a mixed volatile oil; or

   mixing *Herba Pogostemonis, Rhizoma Atractylodis, Herba Moslae, Folium Artemisiae Argyi, Flos Caryophylli,* and *Herba Menthae Haplocalycis* according to claim 2, then adding with water and distilling for extraction, to give a total volatile oil; or
   mixing and placing *Herba Pogostemonis, Rhizoma Atractylodis, Herba Moslae, Folium Artemisiae Argyi, Flos Caryophylli*, and *Herba Menthae Haplocalycis* according to claim 2 in a supercritical carbon dioxide extraction device, and extracting volatile oil with supercritical extraction by introducing liquid carbon dioxide, to give the total volatile oil; and

   2) adding natural borneol to the mixed volatile oil or total volatile oil, heating to dissolve the natural borneol, to give the pharmaceutical volatile oil, or
   adding the natural borneol to a solvent at 50-60°C and stirring to dissolve, cooling down to room temperature, then adding to the mixed volatile oil or total volatile oil, to give the pharmaceutical volatile oil.

**5.** The antibacterial and antiviral pharmaceutical volatile oil according to claim 4, wherein,

the preparation methods of the volatile oil of each of the raw materials are as follows:

*Herba Pogostemonis* is added with 5-8 times its own weight in water, and soaked overnight, followed by extracting by steam distillation for 4-8h;
*Rhizoma Atractylodis* is added with 5-8 times its own weight in water and soaked for 20-60 min, followed by extracting by steam distillation for 4-6h;
*Herba Moslae* is added with 5-8 times its own weight in water and soaked for 1-3h, followed by extracting by steam distillation for 2-4h;
*Folium Artemisiae Argyi* is added with 6-10 times its own weight in water and soaked for 4-8h, followed by extracting by steam distillation for 4-6h;
*Flos Caryophylli* is added with 5-8 times its own weight in water and soaked overnight, followed by extracting by steam distillation for 4-8h;
*Herba Menthae Haplocalycis* is added with 5-8 times its own weight in water and soaked for 1-3h, followed by extracting by steam distillation for 4-6h;

a preparation method for the total volatile oil is as follows:
*Herba Pogostemonis, Rhizoma Atractylodis, Herba Moslae, Folium Artemisiae Argyi, Flos Caryophylli*, and *Herba Menthae Haplocalycis* are mixed and added with 5-10 times their own weight in water, and soaked for 1-8h, followed by extracting by steam distillation for 2-8h; or *Herba Pogostemonis, Rhizoma Atractylodis, Herba Moslae, Folium Artemisiae Argyi, Flos Caryophylli*, and *Herba Menthae Haplocalycis* are mixed and placed in a supercritical carbon dioxide extraction device, followed by extraction of the volatile oil by introducing liquid carbon dioxide, with extraction pressure of 20-40 Mpa, extraction temperature of 40-70 °C, separation temperature of 30-60 °C and extraction time of 0.5-10h.

**6.** An antibacterial and antiviral pharmaceutical aromatic water, wherein: *Herba Pogostemonis, Rhizoma Atractylodis, Herba Moslae, Folium Artemisiae Argyi, Flos Caryophylli*, and *Herba Menthae Haplocalycis* according to claim 1 are mixed, added with 5-12 times their own weight in water, soaked overnight and then distilled, to give the pharmaceutical aromatic water.

**7.** Use of the pharmaceutical composition according to claim 1, 2 or 3, or the pharmaceutical volatile oil according to claim 4 or 5, or the pharmaceutical aromatic water according to claim 6, in preparing a medicament for anti-bacteria, anti-virus, clearing heat, reducing fever and/or treating a respiratory disease.

**8.** The use according to claim 7, wherein the dosage form of the medicament includes a spray, distillate medicinal water, aerosol, dropping pill, soft capsule, nasal drop or preparation for atomization inhalation.

**9.** The use according to claim 8, wherein

a preparation method for the spray is as follows:

the pharmaceutical volatile oil is diluted with a diluent, stirred well, and filtrated to give the spray; or
natural borneol and the pharmaceutical excipient are added to a solvent at 50-60 °C, stirred to dissolve, and cooled down to room temperature, and then the mixed volatile oil or the total volatile oil is added thereto, diluted with a diluent, stirred well and filtrated to give the spray;

a preparation method for the distillate medicinal water is as follows:
the pharmaceutical excipient is added to the pharmaceutical aromatic water and stirred well, to give the distillate medicinal water;
a preparation method for the aerosol is as follows:

the pharmaceutical volatile oil is diluted with a diluent, stirred well, filtrated, filled in a pressure container, capped and filled with a propellant to give the aerosol; or
natural borneol and the pharmaceutical excipient are added to a solvent at 50-60 °C, stirred to dissolve, and cooled down to room temperature, and then the mixed volatile oil or the total volatile oil is added thereto, diluted with a diluent, stirred well, filtrated, filled in a pressure container, capped and filled with a propellant, to give the aerosol;

a preparation method for the dropping pill is as follows:
the pharmaceutical volatile oil is prepared into volatile oil microcapsule powder by microencapsulation technology, and mixed with a dropping pill matrix, to give the dropping pill;
a preparation method for the soft capsule is as follows:
the pharmaceutical volatile oil is encapsulated with a soft capsule material to give the soft capsule;
a preparation method for the nasal drop is as follows:
the pharmaceutical volatile oil is dissolved in an anhydrous ethanol solution of a gel matrix material, added with a diluent, stirred well, and dried under reduced pressure to remove anhydrous ethanol, to give a oleogel nasal drop;
a preparation method for the preparations for atomization inhalation is as follows:
the pharmaceutical volatile oil is diluted with a diluent, stirred well, filtrated and filled.

10. The use according to claim 9, wherein

the solvent is any one of medium chain triglyceride, 1-98% ethanol, anhydrous ethanol, propylene glycol, glycerol or edible oils;
the diluent is any one of medium chain triglycerides, 1-98% ethanol, anhydrous ethanol, propylene glycol, glycerol or edible oils;
the propellant is nitrogen gas, carbon dioxide, heptafluoropropane, tetrafluoroethane, 1,3,3,3-tetrafluoropropene, 2,3,3,3-tetrafluoropropene or compressed air.

11. Use of the pharmaceutical composition according to claim 1, 2 or 3, or the pharmaceutical volatile oil according to claim 4 or 5, or the pharmaceutical aromatic water according to claim 6 in preparing an anti-SARS-CoV-2 medicament.

Figure 1

Figure 2

Figure 3

Figure 4

Figure 5

Figure 6

Figure 7

Figure 8

## INTERNATIONAL SEARCH REPORT

| International application No. |
|---|
| **PCT/CN2021/090137** |

**A. CLASSIFICATION OF SUBJECT MATTER**

A61K 36/61(2006.01)i; A61K 31/045(2006.01)i; A61P 31/12(2006.01)i; A61P 31/14(2006.01)i; A61P 31/04(2006.01)i; A61P 11/00(2006.01)i; A61P 11/02(2006.01)i; A61P 29/00(2006.01)i; A61P 37/08(2006.01)i; A61K 9/12(2006.01)i; A61K 9/72(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, CNMED, TWABS, MOABS, HKABS, TWMED, CNTXT, TWTXT, CJFD, MEDNPL, CSCD, SIPONPL, ZYNPL, DWPI, SIPOABS, CPEA, USTXT, WOTXT, EPTXT, CATXT, JPTXT, VEN, 中国药物专利数据库(网页版), 药品标准库(E 药全库和国家新药注册数据), CNKI: 藿香, 香薷, 苍术, 艾叶, 丁香, 薄荷, 冰片, 抗菌, 抗病毒, 新冠病毒, 新型冠状病毒, 清热, 退热, 呼吸系统疾病, 鼻, 咽喉, 气管, 支气管, 肺, 抗炎, 止咳, 抗哮喘, 化痰, 过敏性鼻炎, 喷雾剂, 露剂, 气雾剂, 滴丸, 软胶囊, 滴鼻剂, 雾化剂, Herba agastaches, Herba Pogostemonis, patchouli, Herba Elsholtziae, Herba Moslae, Chinese mosla herb, Rhizoma Atractylodis, Folium Artemisiae Argyi, Artemisia leaves, Flos Caryophylli, clove, Herba Menthae, mint, borneol, antibacterial, antiviral, new coronavirus, SARS COV 2, clearing heat, reducing fever, respiratory diseases, nose, pharynx, larynx, trachea, bronchus, lung, anti-inflammatory, cough suppressant, anti-asthmatic, reducing phlegm, allergic rhinitis, spray, lotion, aerosol, drop pill, soft capsule, nose drop, atomizer liquid

**C. DOCUMENTS CONSIDERED TO BE RELEVANT**

| Category* | Citation of document, with indication, where appropriate, of the relevant passages | Relevant to claim No. |
|---|---|---|
| PX | CN 111494469 A (YUNNAN BAIYAO GROUP CO., LTD.) 07 August 2020 (2020-08-07) claims 1-11, description paragraph 76, paragraph 310 | 1-11 |
| Y | CN 101104065 A (NANJING UNIVERSITY OF CHINESE MEDICINE) 16 January 2008 (2008-01-16) claims 1-5, description pages 1-15 | 1-11 |
| Y | CN 103585540 A (YUNNAN UNIVERSITY OF CHINESE MEDICINE) 19 February 2014 (2014-02-19) claims 1-3, description pages 1-3 | 1-11 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 July 2021** | **02 August 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

# EP 4 144 358 A1

## INTERNATIONAL SEARCH REPORT

| International application No. |
| --- |
| **PCT/CN2021/090137** |

### C.  DOCUMENTS CONSIDERED TO BE RELEVANT

| Category* | Citation of document, with indication, where appropriate, of the relevant passages | Relevant to claim No. |
| --- | --- | --- |
| Y | 明溪 等 (MING, Xi et al.). "苍艾香熏油体外抑菌作用研究 (Research of Extra-Corporeal Bacteriostasis of Incense-Fumigating oil of Rhizoma Atractylodis & Folium Artemisiae Argyi)" 云南中医学院学报 *(Journal of Yunnan University of Traditional Chinese Medicine)*, Vol. 31, No. 01, 20 February 2008 (2008-02-20), pages 34-35 and 41 | 1-11 |
| Y | 明溪 等 (MING, Xi et al.). "苍艾香熏油体外抑菌作用研究 (Research of Extra-Corporeal Bacteriostasis of Incense-Fumigating oil of Rhizoma Atractylodis & Folium Artemisiae Argyi)" 云南中医学院学报 *(Journal of Yunnan University of Traditional Chinese Medicine)*, Vol. 34, No. 01, 20 February 2011 (2011-02-20), pp. 10-13 | 1-11 |
| Y | 明溪 等 (MING, Xi et al.). "苍艾香熏油的制备工艺研究 (Preparation Technology of Cang Ai Essential Oil)" 云南中医中药杂志 *(Yunnan Journal of Traditional Chinese Medicine and Materia Medica)*, Vol. 35, No. 05, 31 May 2014 (2014-05-31), pp. 77-78 | 1-11 |
| Y | 齐佳龙 等 (QI, Jialong et al.). "基于文献的含挥发性成分中药防治新型冠状病毒肺炎作用探究 (non-official translation: Literature-Based Research on the Prevention and Treatment of COVID-19 Using Traditional Chinese Medicine Containing Volatile Components)" 中医文献杂志 *(Journal of Traditional Chinese Medical Literature)*, Vol. 38, No. 02, 25 April 2020 (2020-04-25), pp. 18-20 | 7-11 |

Form PCT/ISA/210 (second sheet) (January 2015)

**INTERNATIONAL SEARCH REPORT**
Information on patent family members

International application No.

**PCT/CN2021/090137**

| Patent document cited in search report | | | Publication date (day/month/year) | Patent family member(s) | | | Publication date (day/month/year) |
|---|---|---|---|---|---|---|---|
| CN | 111494469 | A | 07 August 2020 | None | | | |
| CN | 101104065 | A | 16 January 2008 | CN | 101104065 | B | 19 May 2010 |
| CN | 103585540 | A | 19 February 2014 | CN | 103585540 | B | 17 June 2015 |

Form PCT/ISA/210 (patent family annex) (January 2015)